# EUROPÄISCHE PATENTSCHRIFT

(11) **EP 2 558 462 B1**
(45) Veröffentlichungstag und Bekanntmachung des Hinweises auf die Patenterteilung: **22.10.2014**
(21) Anmeldenummer: 11714317.2
(22) Anmeldetag: 14.04.2011
(51) Int. Cl.: C07D 471/04, A61K 31/444, A61P 9/00

(54) **PYRIDYL-VINYL-PYRAZOLO-CHINOLINE ALS PAR1-INHIBITOREN**
Pyridyl-vinyl-pyrazolo-chinolines as PAR1 inhibitors
Pyridyl-vinyle-pyrazolo-chinolines comme inhibiteurs de PAR1

(30) Priorität: 16.04.2010 EP 10305395
(43) Veröffentlichungstag der Anmeldung: 20.02.2013
(73) Patentinhaber: SANOFI, 75008 Paris (FR)
(72) Erfinder: SCHOENAFINGER, Karl, 65926 Frankfurt am Main (DE); STEINHAGEN, Henning, 65926 Frankfurt am Main (DE); SCHEIPER, Bodo, 65926 Frankfurt am Main (DE); HEINELT, Uwe, 65926 Frankfurt am Main (DE); WEHNER, Volkmar, 65926 Frankfurt am Main (DE); HERRMANN, Matthias, 65926 Frankfurt am Main (DE); MAUGER, Jacques, Oro Valley Arizona 85755-1965 (US); SAFAR, Pavel, Oro Valley Arizona 85755-1965 (US)
(74) Vertreter: Venne-Dunker, Sabine
(86) Internationale Anmeldenummer: PCT/EP2011/055964
(87) Internationale Veröffentlichungsnummer: WO 2011/128420

(56) Entgegenhaltungen:
- WO-A2-2009/124103
- CHACKALAMANNIL S ET AL: "Thrombin receptor (PAR-1) antagonists as novel antithrombotic agents", EXPERT OPINION ON THERAPEUTIC PATENTS, INFORMA HEALTHCARE, GB LNKD- DOI:10.1517/13543776.16.4.493, Bd. 16, Nr. 4, 1. April 2006 (2006-04-01), Seiten 493-505, XP002415653, ISSN: 1354-3776
- CHEBANOV V. A. ET AL: "Tuning of Chemo- and Regioselectivities in Multicomponent Condensations of 5-Aminopyrazoles, Dimedone, and Aldehydes", JOURNAL OF ORGANIC CHEMISTRY, Bd. 73, Nr. 13, 2008, Seiten 5110-5118, XP002589214,
- JAIRO QUIROGA ET AL: "Synthesis of 4-Aryl-4,7,8,9-tetrahydro-6H-pyrazolo[3,4- b]quinolin-5-ones", JOURNAL OF HETEROCYCLIC CHEMISTRY, WILEY-BLACKWELL PUBLISHING, INC, US LNKD- DOI:10.1002/JHET.5570350313, Bd. 35, Nr. 3, 1. Mai 1998 (1998-05-01), Seiten 575-578, XP009135322, ISSN: 0022-152X [gefunden am 2009-03-11]

## Beschreibung

Die Erfindung betrifft neue Verbindungen der Formel I, 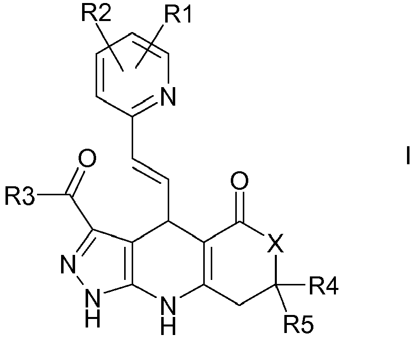 wobei R1, R2, R3, R4, R5 und X die unten bezeichnete Bedeutung haben. Die Verbindungen der Formel I haben antithrombotische Aktivität und inhibieren insbesondere den Protease-aktivierten Rezeptor 1 (PAR1). Die Erfindung betrifft weiterhin Verfahren zur Herstellung der Verbindungen der Formel I und deren Verwendung als Arzneimittel.

Der Protease-aktivierte Rezeptor 1 (PAR1) ist ein Thrombin-Rezeptor, der zur Klasse der G-Protein-gekoppelten Rezeptoren (GPCR) gehört. Das Gen für PAR1 liegt auf Chromosom 5q13, besteht aus zwei Exonen und deckt eine Region von etwa 27 kb ab. PAR1 wird unter anderem in Endothelzellen, glatten Muskelzellen, Fibroblasten, Neuronen und humanen Blutplättchen exprimiert. Auf Blutplättchen ist PAR1 ein wichtiger Rezeptor der Signalübertragung welcher an der Initiation der Aggregation von Blutplättchen beteiligt ist. Die Aktivierung der PARs erfolgt über die proteolytische Abspaltung eines Teils des N-Terminus der PARs, wodurch eine neue N-terminale Sequenz freigelegt wird, die dann den Rezeptor aktiviert (M. D. Hollenberg et al., Pharmacol. Rev. 54:203-217, 2002).

Die Blutgerinnung ist ein für das Überleben von Säugetieren wesentlicher Vorgang der Kontrolle des Blutstroms. Der Vorgang der Gerinnung und der nachfolgenden Auflösung des Gerinnsels nach erfolgter Wundheilung setzt nach einer Gefäßschädigung ein und lässt sich in vier Phasen einteilen:
1. Die Phase der vaskulären Konstriktion: Hierdurch wird der Blutverlust in geschädigte Areale vermindert.
2. Die nächste Phase ist die der Plättchenadhäsion an das freigelegte Kollagen im Subendothel. Diese primäre Adhäsion an die Matrix aktiviert die Plättchen, die daraufhin verschiedene Aktivatoren sekretieren, die zur Verstärkung der Aktivierung führen. Diese Aktivatoren stimulieren zudem die weitere Rekrutierung neuer Plättchen zum Ort der Gefäßschädigung und fördern die Plättchenaggregation. Die Plättchen aggregieren an der Stelle des Gefäßwandschadens und bilden ein noch lockeres Plättchengerinnsel. Weiterhin führt die Aktivierung der Plättchen zur Präsentation von Phosphatidylserin und Phosphatidylinositol entlang der Zellmembranoberflächen. Die Exposition dieser Phospholipide ist zur Bindung und Aktivierung von Multienzymkomplexen der Blutgerinnungskaskade essentiell.
3. Das anfänglich noch lockere Plättchenaggregat wird durch Fibrin vernetzt. Wenn der Thrombus lediglich Plättchen und Fibrin enthält, handelt es sich um einen weißen Thrombus. Sind zusätzlich rote Blutkörperchen vorhanden, handelt es sich um einen roten Thrombus.
4. Nach Wundheilung wird der Thrombus durch die Einwirkung des Proteins Plasmin aufgelöst.

Zwei alternative Wege führen zur Bildung eines Fibringerinnsels, der intrinsische und der extrinsische Weg. Diese Wege werden durch unterschiedliche Mechanismen eingeleitet, in späterer Phase konvergieren sie jedoch zu einer gemeinsamen Wegstrecke der Gerinnungskaskade. Die Bildung eines roten Thrombus oder eines Gerinnsels auf dem Boden einer Gefäßwandabnormität ohne Wunde ist das Resultat des intrinsischen Weges. Die Fibringerinnselbildung als Antwort auf einen Gewebsschaden oder eine Verletzung ist das Resultat des extrinsischen Weges. Beide Wege beinhalten eine größere Anzahl von Proteinen, die als Gerinnungsfaktoren bekannt sind.

Der intrinsische Weg erfordert die Gerinnungsfaktoren VIII, IX, X, XI und XII sowie Präkallekrein, hochmolekulares Kininogen, Calciumionen und Phospholipide aus Plättchen. Jedes dieser Proteine führt zur Aktivierung des Faktors X. Der intrinsische Weg wird eingeleitet, wenn Präkallekrein, hochmolekulares Kininogen Faktor XI und XII an eine negativ geladene Oberfläche binden. Dieser Moment wird als Kontaktphase bezeichnet. Die Exposition gegenüber einem Gefäßwandkollagen ist der primäre Stimulus der Kontaktphase. Resultat der Vorgänge der Kontaktphase ist die Umwandlung von Präkallekrein in Kallekrein, das wiederum den Faktor XII aktiviert. Faktor Xlla hydrolysiert weiteres Präkallekrein zu Kallekrein, so dass eine Aktivierung die Folge ist. Mit zunehmender Aktivierung von Faktor XII kommt es zur Aktivierung des Faktors XI, der zu einer Freisetzung von Bradykinin, einem Vasodilatator führt. Dadurch kommt es zur Beendigung der initialen Phase der Vasokonstriktion. Bradykinin entsteht aus dem hochmolekularen Kininogen. In Anwesenheit von Ca²⁺-Ionen aktiviert der Faktor Xla den Faktor IX. Faktor IX ist ein Proenzym, das Vitamin-K abhängige, γ-Karboxiglutamat (GLA)-Reste enthält. Die Serinproteaseaktivität kommt nach Bindung von Ca²⁺-Ionen an diese GLA-Reste zum Tragen. Mehrere der Serinproteasen der Blutgerinnungskaskade (Faktoren II, VII, IX und X) enthalten derartige Vitamin-K-abhängige GLA-Reste. Faktor IXa spaltet den Faktor X und führt zur Aktivierung zum Faktor Xa. Voraussetzung für die Bildung von Faktor IXa ist die Bildung eines Proteasekomplexes aus Ca²⁺-Ionen und den Faktoren VIIIa, IXa und X an der Oberfläche aktivierter Plättchen. Eine der Reaktionen aktivierter Plättchen ist die Präsentation von Phosphatidylserin und Phosphatidylinositol entlang der Oberflächen. Die Exposition dieser Phospholipide macht erst die Bildung des Proteasekomplexes möglich. Faktor VIII hat in diesem Vorgang die Funktion eines Rezeptors für die Faktoren IXa und X. Faktor VIII stellt daher einen Cofaktor in der Gerinnungskaskade dar. Die Aktivierung des Faktors VIII mit Bildung des Faktors VIIIa, dem eigentlichen Rezeptor, bedarf nur einer minimalen Menge von Thrombin. Mit Zunahme der Konzentration von Thrombin wird der Faktor VIIIa schließlich durch Thrombin weiter gespalten und inaktiviert. Diese duale Aktivität des Thrombins in Bezug zum Faktor VIII führt zu einer Selbstbegrenzung der Proteasekomplexbildung und damit zu einer Eingrenzung der Blutgerinnung.

Bei der Aktivierung von humanen Blutplättchen durch Thrombin spielen PAR1 und PAR4 eine zentrale Rolle; die Aktivierung dieser Rezeptoren führt in Blutplättchen zu morphologischen Veränderungen, Freisetzung von ADP und Aggregation der Blutplättchen (S. Brass, Nature 413:26-27, 2001).

Inhibitoren von PAR1 werden beispielsweise in EP 1391451, EP 1391452, US 6063847, US 2004/152736, US 2004/176418, US 2004/192753, US 2005/267155, US 2006/063847, US 2006/079684, US 2007/149518, US 2007/232635, US 6326380, WO 99/26943, WO 01/96330, WO 03/089428, WO 2006/076564, WO 2006/105217 und WO 2008/042422 beschrieben.

Es wurde gefunden, dass die Verbindungen der Formel I eine hohe spezifische Inhibierung des Protease-aktivierten Rezeptors 1 zeigen. Die Verbindungen der Formel I eignen sich daher für die prophylaktische als auch für die therapeutische Anwendung am Menschen, die an Erkrankungen leiden, die mit Thrombosen, Embolien, Hyperkoagulabilität oder fibrotischen Veränderungen einhergehen. Beispiele solcher Erkrankungen sind Thrombose, tiefe Venen-thrombose, Lungenembolien, Gehirninfarkt, Herzinfarkt, Bluthochdruck, entzündliche Erkrankungen, Rheuma, Asthma, Glomerulonephritis oder Osteoporose. Die Verbindungen der Formel I können zur Sekundär-Prävention eingesetzt werden und eignen sich sowohl für eine akute als auch für eine Langzeittherapie. Die Verbindungen der Formel I sind auch in Kombination mit Wirkstoffen einsetzbar, die über andere antithrombotische Prinzipien als PAR1 wirken.

Die Erfindung betrifft daher eine Verbindung der Formel I, 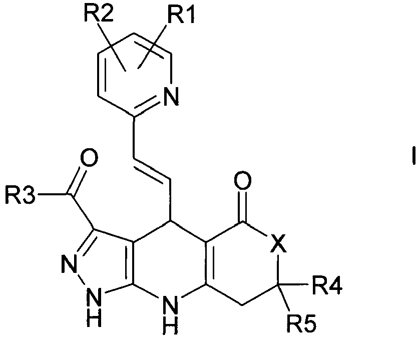 und/oder alle stereoisomeren oder tautomeren Formen der Verbindung der Formel I und/oder Gemische dieser Formen in jedem Verhältnis, und/oder ein physiologisch verträgliches Salz der Verbindung der Formel I, wobei
R1 und R2 gleich oder verschieden sind und unabhängig voneinander für Wasserstoff, -(C₁-C₆)-Alkyl, -O-(C₁-C₆)-Alkyl, Aryl, Halogen oder Hetaryl ausgewählt aus der Gruppe Thienyl, Pyrrolyl, Imidazolyl, Pyrazolyl, Pyridyl, Pyrimidinyl, Pyrazinyl oder Pyridazinyl stehen, wobei Alkyl, Aryl und Hetaryl jeweils unsubstituiert oder einfach, zweifach oder dreifach unabhängig voneinander durch F, Cl, Br, CN, -(C₁-C₄)-Alkyl, -O-(C₁-C₄)-Alkyloder -CF₃ substituiert sind,
R3 für -OH, -O-(C₁-C₈)-Alkyl, -O-(C₁-C₆)-Alkylen-NH-C(O)-O-(C₁-C₄)-Alkyl, -O-(C₁-C₆)-Alkylen-NH₂, -NH₂, -NH-(C₁-C₈)-Alkyl, -NH-(C₁-C₆)-Alkylen-Aryl, -NH-(C₁-C₈)-Alkylen-O-(C₁-C₄)-Alkyl, -NH-(C₁-C₈)-Alkyl-OH, -NH-(C₁-C₆)-Alkylen-NH-C(O)-O-(C₁-C₄)-Alkyl, -NH-(C₁-C₆)-Alkylen-NH₂, -NH-(C₀-C₆)-Alkylen-(C₃-C₇)-Cycloalkyl, -NH-(C₀-C₆)-Alkylen-Hetaryl oder -N((C₁-C₄)-Alkyl)₂ steht, wobei Aryl, Cycloalkyl und Hetaryl jeweils unsubstituiert oder einfach, zweifach oder dreifach unabhängig voneinander durch F, Cl, -OH, -(C₁-C₄)-Alkyl, -(C₁-C₄)-Alkylen-NH₂, -(C₁-C₄)-Alkylen-OH oder -O-(C₁-C₄)-Alkyl substituiert sind;
R4 und R5 gleich oder verschieden sind und unabhängig voneinander für Wasserstoff oder -(C₁-C₆)-Alkyl stehen, oder
R4 und R5 zusammen mit dem C-Atom, an das sie gebunden sind, einen (C₃-C₇)-Cycloalkylrest bilden;
X für eine kovalente Bindung, CH₂, CH((C₁-C₆)-Alkyl), C((C₁-C₄)-Alkyl)₂ oder Sauerstoff steht;
wobei unter dem Begriff "Hetaryl" in R3 Ringsysteme verstanden werden mit 4 bis 15 Kohlenstoffatomen, die in ein, zwei oder drei miteinander verbundenen Ringsystemen vorliegen und die je nach Ringgröße ein, zwei, drei oder vier gleiche oder verschiedene Heteroatome aus der Reihe Sauerstoff, Stickstoff und Schwefel enthalten oder wobei Hetaryl für einen Oxetanylrest steht.

Die Erfindung betrifft weiterhin eine Verbindung der Formel I, und/oder alle stereoisomeren oder tautomeren Formen der Verbindung der Formel I und/oder Gemische dieser Formen in jedem Verhältnis, und/oder ein physiologisch verträgliches Salz der Verbindung der Formel I, wobei
R1 und R2 gleich oder verschieden sind und unabhängig voneinander für Wasserstoff, -(C₁-C₆)-Alkyl, -O-(C₁-C₆)-Alkyl, Aryl, Halogen oder Hetaryl ausgewählt aus der Gruppe Thienyl, Pyrrolyl, Imidazolyl, Pyrazolyl, Pyridyl, Pyrimidinyl, Pyrazinyl oder Pyridazinyl stehen, wobei Alkyl, Aryl und Hetaryl jeweils unsubstituiert oder einfach, zweifach oder dreifach unabhängig voneinander durch F, Cl, Br, CN, -(C₁-C₄)-Alkyl, -O-(C₁-C₄)-Alkyl oder -CF₃ substituiert sind;

R3 für -O-(C₁-C₆)-Alkyl, -O-(C₁-C₆)-Alkylen-NH-C(O)-O-(C₁-C₄)-Alkyl, -O-(C₁-C₆)-Alkylen-NH₂, -NH-(C₁-C₆)-Alkyl, -NH-(C₁-C₆)-Alkylen-Aryl, -NH-(C₁-C₆)-Alkylen-(C₃-C₇)-Cycloalkyl, -NH-(C₁-C₆)-Alkylen-Hetaryl oder -N((C₁-C₄)-Alkyl)₂ steht, wobei Aryl, Cycloalkyl und Hetaryl jeweils unsubstituiert oder einfach, zweifach oder dreifach unabhängig voneinander durch F, Cl, -OH, -(C₁-C₄)-Alkylen-NH₂, -(C₁-C₄)-Alkylen-OH oder -(C₁-C₄)-Alkyl substituiert sind;

R4 und R5 gleich oder verschieden sind und unabhängig voneinander für Wasserstoff oder -(C₁-C₆)-Alkyl stehen, oder
R4 und R5 zusammen mit dem C-Atom, an das sie gebunden sind, (C₃-C₆)-Cycloalkyl bilden;
X für eine kovalente Bindung, CH₂, CH((C₁-C₄)-Alkyl), C((C₁-C₄)-Alkyl)₂ oder Sauerstoff steht;
wobei unter dem Begriff "Hetaryl" in R3 Ringsysteme verstanden werden mit 4 bis 15 Kohlenstoffatomen, die in ein, zwei oder drei miteinander verbundenen Ringsystemen vorliegen und die je nach Ringgröße ein, zwei, drei oder vier gleiche oder verschiedene Heteroatome aus der Reihe Sauerstoff, Stickstoff oder Schwefel enthalten oder wobei Hetaryl für einen Oxetanylrest steht.

Die Erfindung betrifft weiterhin eine Verbindung der Formel la, und/oder alle stereoisomeren oder tautomeren Formen der Verbindung der Formel la und/oder Gemische dieser Formen in jedem Verhältnis, und/oder ein physiologisch verträgliches Salz der Verbindung der Formel Ia, 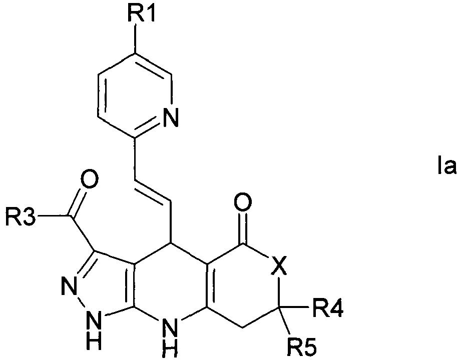 wobei
R1 für Wasserstoff, -(C₁-C₄)-Alkyl, Phenyl, Cl, Br oder Hetaryl steht, wobei Hetaryl ausgewählt ist aus der Gruppe Imidazolyl, Pyrazinyl, Pyrazolyl, Pyridazinyl, Pyridyl, Pyrimidinyl, Pyrrolyl, und Thienyl,
und wobei Phenyl und Hetaryl jeweils unsubstituiert oder einfach oder zweifach unabhängig voneinander durch F, Br, Cl, CN, -(C₁-C₄)-Alkyl, -O-(C₁-C₄)-Alkyl oder-CF₃ substituiert sind;
R3 für -OH, -O-(C₁-C₆)-Alkyl, -O-(C₁-C₆)-Alkylen-NH-C(O)-O-(C₁-C₄)-Alkyl, -O-(C₁-C₆)-Alkylen-NH₂, -NH-(C₁-C₄)-Alkyl, -NH-Benzyl oder -NH-Methylen-(C₃-C₆)-Cycloalkyl steht, wobei Cycloalkyl unsubstituiert oder einfach durch -(C₁-C₄)-Alkyl, -(C₁-C₄)-Alkylen-NH₂ oder -CH₂-OH substituiert ist;
R4 und R5 gleich oder verschieden sind und unabhängig voneinander für Wasserstoff oder -(C₁-C₄)-Alkyl stehen, oder
R4 und R5 zusammen mit dem C-Atom, an das sie gebunden sind, Cyclopropyl, Cyclobutyl, Cyclopentyl oder Cyclohexyl bilden;
X für eine kovalente Bindung, CH₂, C((C₁-C₄)-Alkyl)₂ oder Sauerstoff steht.

Die Erfindung betrifft weiterhin eine Verbindung der Formel la, und/oder alle stereoisomeren oder tautomeren Formen der Verbindung der Formel Ia und/oder Gemische dieser Formen in jedem Verhältnis, und/oder ein physiologisch verträgliches Salz der Verbindung der Formel Ia, wobei
R1 für -(C₁-C₄)-Alkyl, Phenyl, Cl oder Br steht, wobei Phenyl unsubstituiert oder einfach, zweifach oder dreifach unabhängig voneinander durch F, Cl, Br, CN, -(C₁-C₄)-Alkyl, -O-(C₁-C₄)-Alkyl oder -CF₃ substituiert ist;
R3 für -OH, -O-(C₁-C₄)-Alkyl, -O-(C₁-C₆)-Alkylen-NH-C(O)-O-(C₁-C₄)-Alkyl, -O-(C₁-C₆)-Alkylen-NH₂, -NH-(C₁-C₃)-Alkyl oder -NH-Methylen-(C₃-C₆)-Cycloalkyl steht, wobei Cycloalkyl unsubstituiert oder einfach durch -(C₁-C₄)-Alkyl, -(C₁-C₄)-Alkylen-NH₂ oder -CH₂-OH substituiert ist;
R4 und R5 gleich oder verschieden sind und unabhängig voneinander für Wasserstoff oder-(C₁-C₄)-Alkyl stehen, oder
R4 und R5 zusammen mit dem C-Atom, an das sie gebunden sind, Cyclobutyl oder Cyclopentyl bilden;
X für eine kovalente Bindung, CH₂, C((C₁-C₄)-Alkyl)₂ oder Sauerstoff steht.

Die Erfindung betrifft weiterhin eine Verbindung der Formel I oder la, und/oder alle stereoisomeren oder tautomeren Formen der Verbindung der Formel I oder la und/oder Gemische dieser Formen in jedem Verhältnis, und/oder ein physiologisch verträgliches Salz der Verbindung der Formel I oder la, ausgewählt aus folgenden Verbindungen:
4-{((E)-2-[5-(3-Fluor-phenyl)-pyridin-2-yl]-vinyl}-7,7-dimethyl-5-oxo-4,5,6,7,8,9-hexahydro-1H-pyrazolo[3,4-b]chinolin-3-carbonsäure-cyclopropylmethylamid,
7,7-Dimethyl-4-[(E)-2-(6-methyl-pyridin-2-yl)-vinyl]-5-oxo-4,5,6,7,8,9-hexahydro-1H-pyrazolo[3,4-b]chinolin-3-carbonsäure-isopropylamid,
4-{((E)-2-[5-(3-Fluor-phenyl)-pyridin-2-yl]-vinyl}-7,7-dimethyl-5-oxo-4,5,6,7,8,9-hexahydro-1H-pyrazolo[3,4-b]chinolin-3-carbonsäure-isopropylamid,
4-{((E)-2-[5-(3-Fluor-phenyl)-pyridin-2-yl]-vinyl}-6,6-dimethyl-5-oxo-4,5,6,7,8,9-hexahydro-1H-pyrazolo[3,4-b]chinolin-3-carbonsäure-cyclopropylmethylamid,
4-{((E)-2-[5-(3-Fluor-phenyl)-pyridin-2-yl]-vinyl}-7-isopropyl-5-oxo-4,5,6,7,8,9-hexahydro-1H-pyrazolo[3,4-b]chinolin-3-carbonsäure-cyclopropylmethylamid,
4-[((E)-2-(5-Brom-pyridin-2-yl)-vinyl]-7,7-dimethyl-5-oxo-4,5,6,7,8,9-hexahydro-1H-pyrazolo[3,4-b]chinolin-3-carbonsäure-cyclopropylmethylamid,
4-[((E)-2-(5-Bromo-pyridin-2-yl)-vinyl]-7,7-dimethyl-5-oxo-4,5,6,7,8,9-hexahydro-1 H-pyrazolo[3,4-b]chinolin-3-carbonsäure-isopropylamid,
4-{((E)-2-[5-(3-Fluor-phenyl)-pyridin-2-yl]-vinyl}-7-methyl-5-oxo-4,5,6,7,8,9-hexahydro-1 H-pyrazolo[3,4-b]chinolin-3-carbonsäure-isopropylamid,
4-{((E)-2-[5-(3-Fluor-phenyl)-pyridin-2-yl]-vinyl}-7-methyl-5-oxo-4,5,6,7,8,9-hexahydro-1 H-pyrazolo[3,4-b]chinolin-3-carbonsäure-cyclopropylmethylamid,
4-{((E)-2-[5-(3-Fluor-phenyl)-pyridin-2-yl]-vinyl}-7,7-dimethyl-5-oxo-4,5,6,7,8,9-hexahydro-1H-pyrazolo[3,4-b]chinolin-3-carbonsäure-ethylester, und
4-{((E)-2-[5-(3-Fluoro-phenyl)-pyridin-2-yl]-vinyl}-7,7-dimethyl-5-oxo-4,5,6,7,8,9-hexahydro-1H-pyrazolo[3,4-b]chinolin-3-carbonsäure-(2,2-dimethyl-propyl)amid.

Unter dem Begriff "Alkyl", "-(C₁-C₆)-Alkyl" und "-(C₁-C₄)-Alkyl" werden Kohlenwasserstoffreste verstanden, deren Kohlenstoffkette geradkettig oder verzweigt ist und die 1, 2, 3, 4, 5 oder 6 Kohlenstoffatome bzw. 1, 2, 3 oder 4 Kohlenstoffatome enthalten, beispielsweise Methyl, Ethyl, Propyl, Isopropyl, Butyl, Isobutyl, sec-Butyl, tert-Butyl, Pentyl, Isopentyl, Neopentyl, 1-Ethylpropyl, Hexyl, 2,3-Dimethylbutyl oder Neohexyl. Bevorzugt enthält -(C₁-C₆)-Alkyl 1 bis 4 Kohlenstoffatome. "-C₀-Alkylen" ist eine kovalente Bindung. Unter dem Begriff "kovalente Bindung" wird eine Form der chemischen Bindung verstanden und ist als solche für den festen Zusammenhalt von Atomen in vielen chemischen Verbindungen verantwortlich. Kovalente Bindung bilden sich besonders zwischen den Atomen von Nichtmetallen aus.

Unter dem Begriff "-O-Alkyl", "-O-(C₁-C₆)-Alkyl" und "-O-(C₁-C₄)-Alkyl" werden Alkoxyreste verstanden, deren Kohlenstoffkette geradkettig oder verzweigt ist und die 1, 2, 3, 4, 5 oder 6 Kohlenstoffatome bzw. 1, 2, 3 oder 4 Kohlenstoffatome enthalten, wie Methoxy, Ethoxy, Propoxy, Isopropoxy, Butoxy, Isobutoxy, tert-Butoxy, 1-Pentoxy, 2-Pentoxy, 3-Pentoxy, 1-Hexoxy, 2-Hexoxy oder 3-Hexoxy. Bevorzugt enthält-O-(C₁-C₆)-Alkyl 1 bis 4 Kohlenstoffatome.

Unter dem Begriff "-(C₃-C₇)-Cycloalkyl" werden 3-gliedrige bis 7-gliedrige Monocyclen verstanden wie Cyclopropyl, Cyclobutyl, Cyclopentyl, Cyclohexyl oder Cycloheptyl, unter dem Begriff "-(C₃-C₆)-Cycloalkyl" 3-gliedrige bis 6-gliedrige Monocyclen wie Cyclopropyl, Cyclobutyl, Cyclopentyl oder Cyclohexyl.

Unter dem Begriff "Aryl" werden aromatische Kohlenwasserstoffreste verstanden mit 6 bis 14 Kohlenstoffatomen im Ring. Arylreste sind beispielsweise Phenyl, Naphthyl, zum Beispiel 1-Naphthyl, 2-Naphthyl, Anthryl oder Fluorenyl. Phenylreste und Naphthylreste, insbesondere Phenylreste, sind bevorzugte Arylreste.

Unter dem Begriff "Hetaryl" in der Definition von R3 werden Ringsysteme verstanden mit 4 bis 15 Kohlenstoffatomen, die in ein, zwei oder drei miteinander verbundenen Ringsystemen vorliegen und die je nach Ringgröße ein, zwei, drei oder vier gleiche oder verschiedene Heteroatome aus der Reihe Sauerstoff, Stickstoff oder Schwefel enthalten. Beispiele für diese Ringsysteme sind die Reste Acridinyl, Azepinyl, Azetidinyl, Aziridinyl, Benzimidazolyl, Benzofuranyl, Benzothiofuranyl, Benzothiophenyl, Benzoxazolyl, Benzthiazolyl, Benztriazolyl, Benzisoxazolyl, Benzisothiazolyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Decahydrochinolinyl, Dibenzofuranyl, Dibenzothiophenyl, Dihydrofuran[2,3-b]-tetrahydrofuranyl, Dihydrofuranyl, Dioxolyl, Dioxanyl, 2H,6H-1,5,2-Dithiazinyl, Furanyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochinolinyl, Isochromanyl, Isoindazolyl, Isoindolinyl, Isoindolyl, Isothiazolidinyl, 2-Isothiazolinyl, Isothiazolyl, Isoxazolyl, Isoxazolidinyl, 2-Isoxazolinyl, Morpholinyl, Naphthyridinyl, Octahydroisochinolinyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxetanyl, Oxothiolanyl, Phenanthridinyl, Phenanthrolinyl, Phenazinyl, Phenothiazinyl, Phenoxathünyl, Phenoxazinyl, Phthalazinyl, Piperazinyl, Piperidinyl, Pteridinyl, Purinyl, Pyranyl, Pyrazinyl, Pyrazolidinyl, Pyrazolinyl, Pyrazolyl, Pyridazinyl, Pyridooxazolyl, Pyridoimidazolyl, Pyridothiazolyl, Pyridothiophenyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyranyl, Tetrahydropyridinyl, 6H-1,2,5-Thiadiazinyl, 1,2,3-Thiadiazolyl, 1,2,4-Thiadiazolyl, 1,2,5-Thiadiazolyl, 1,3,4-Thiadiazolyl, Thianthrenyl, Thiazolidinyl, Thiazolinyl, Thiazolyl, Thienyl, Thienoimidazolyl, Thienooxazolyl, Thienopyrrolyl, Thienopyridinyl, Thienothiazolyl, Thienothiophenyl, Thiomorpholinyl, Triazinyl, 1,2,3-Triazolyl, 1,2,4-Triazolyl, 1,2,5-Triazolyl, 1,3,4-Triazolyl oder Xanthenyl.

Unter dem Begriff "Halogen" wird Fluor, Chlor, Brom oder Iod verstanden, bevorzugt sind Fluor, Chlor oder Brom, insbesondere Fluor oder Chlor.

Unter dem Begriff "X für eine kovalente Bindung steht" wird der folgende Teilring der Formel I oder la verstanden:

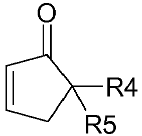

Unter dem Begriff "X für CH₂ steht" wird der folgende Teilring der Formel I oder la verstanden:

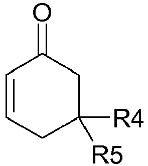

Unter dem Begriff "X für CH((C₁-C₆)-Alkyl) steht" wird der folgende Teilring der Formel I oder la verstanden, worin Y -(C₁-C₆)-Alkyl bedeutet:

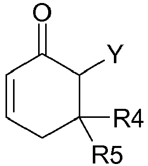

Unter dem Begriff "X für C((C₁-C₄)-Alkyl)₂ steht" wird der folgende Teilring der Formel I oder la verstanden, worin Y -((C₁-C₄)-Alkyl)₂ bedeutet: 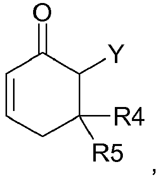 der auch durch die folgende Formel dargestellt werden kann, worin die beiden Gruppen Y jeweils -(C₁-C₄)-Alkyl) bedeuten.

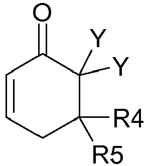

Unter dem Begriff "X für N-R6 steht" wird der folgende Teilring der Formel I oder la verstanden:

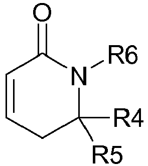

Unter dem Begriff "X für O steht", das heißt "X für Sauerstoff oder ein Sauerstoffatom steht", wird der folgende Teilring der Formel I oder la verstanden:

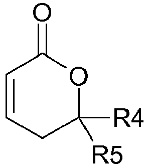

Funktionelle Gruppen in den Verbindungen der Formeln I und la und den zu deren Herstellung verwendeten Intermediaten, beispielsweise Aminogruppen oder Carboxylgruppen, können durch geeignete Schutzgruppen maskiert werden. Geeignete Schutzgruppen für Aminofunktionen sind beispielsweise die tert-Butoxycarbonyl-, die Benzyloxycarbonyl- oder die Phthaloylgruppe sowie die Trityl- oder die Tosylschutzgruppe. Geeignete Schutzgruppen für die Carboxylfunktion sind beispielsweise Alkyl-, Aryl- oder Arylalkylester. Schutzgruppen können durch wohlbekannte oder hier beschriebene Techniken eingeführt und entfernt werden (siehe Greene, T. W., Wuts, P.G.M., Protective Groups in Organic Synthesis (1999), 3rd Ed., Wiley-Interscience; oder Kocienski, P. J., Protecting Groups (2004), 3rd Ed., Thieme Verlag). Der Begriff Schutzgruppe kann auch entsprechende polymergebundene Schutzgruppen umfassen.

In einer Ausführungsform der Erfindung sind R1 und R2 in den Verbindungen der Formel I gleich oder verschieden und stehen unabhängig voneinander für Wasserstoff, -(C₁-C₆)-Alkyl, -O-(C₁-C₆)-Alkyl, Aryl, Halogen oder Hetaryl ausgewählt aus der Gruppe Thienyl, Pyrrolyl, Imidazolyl, Pyrazolyl, Pyridyl, Pyrimidinyl, Pyrazinyl oder Pyridazinyl, wobei Alkyl, Aryl und Hetaryl jeweils unsubstituiert oder einfach, zweifach oder dreifach unabhängig voneinander durch F, Cl, Br, CN, -(C₁-C₄)-Alkyl, - O-(C₁-C₄)-Alkyl oder -CF₃ substituiert sind. In einer anderen Ausführungsform sind R1 und R2 in den Verbindungen der Formel I gleich oder verschieden und stehen unabhängig voneinander für Wasserstoff, -(C₁-C₆)-Alkyl, -O-(C₁-C₆)-Alkyl, Aryl, Halogen oder Hetaryl ausgewählt aus der Gruppe Thienyl, Pyrrolyl, Imidazolyl, Pyrazolyl, Pyridyl, Pyrimidinyl, Pyrazinyl oder Pyridazinyl, wobei Aryl und Hetaryl jeweils unsubstituiert oder einfach, zweifach oder dreifach unabhängig voneinander durch F, Cl, Br, -(C₁-C₄)-Alkyl, -O-(C₁-C₄)-Alkyl, -OCF₃ oder -CF₃ substituiert sind. In einer anderen Ausführungsform sind R1 und R2 in den Verbindungen der Formel I gleich oder verschieden und stehen unabhängig voneinander für Wasserstoff, -(C₁-C₆)-Alkyl, -O-(C₁-C₆)-Alkyl, Aryl, Halogen oder Hetaryl ausgewählt aus der Gruppe Thienyl, Pyrrolyl, Imidazolyl, Pyrazolyl, Pyridyl, Pyrimidinyl, Pyrazinyl oder Pyridazinyl, wobei Aryl und Hetaryl jeweils unsubstituiert oder einfach, zweifach oder dreifach unabhängig voneinander durch F, Cl, Br, -(C₁-C₄)-Alkyl oder -CF₃ substituiert sind. In einer anderen Ausführungsform sind R1 und R2 in den Verbindungen der Formel I gleich oder verschieden und stehen unabhängig voneinander für Wasserstoff, -(C₁-C₆)-Alkyl, Aryl, Halogen oder Hetaryl ausgewählt aus der Gruppe Thienyl, Pyrrolyl, Imidazolyl, Pyrazolyl, Pyridyl, Pyrimidinyl, Pyrazinyl oder Pyridazinyl, wobei Aryl und Hetaryl jeweils unsubstituiert oder einfach, zweifach oder dreifach unabhängig voneinander durch F, Cl, Br, -(C₁-C₄)-Alkyl oder -CF₃ substituiert sind. In einer anderen Ausführungsform sind R1 und R2 in den Verbindungen der Formel I gleich oder verschieden und stehen unabhängig voneinander für Wasserstoff, -(C₁-C₆)-Alkyl, Phenyl oder Halogen, wobei Phenyl unsubstituiert oder einfach, zweifach oder dreifach unabhängig voneinander durch F, Cl, Br, -(C₁-C₄)-Alkyl oder -CF₃ substituiert ist.

In einer Ausführungsform der Erfindung steht eine der Gruppen R1 und R2 in den Verbindungen der Formel I für Wasserstoff, -(C₁-C₆)-Alkyl, -O-(C₁-C₆)-Alkyl oder Halogen, in einer anderen Ausführungsform für Wasserstoff, -(C₁-C₆)-Alkyl oder Halogen, in einer anderen Ausführungsform für Wasserstoff oder Halogen, und die andere der Gruppen R1 und R2 steht in einer Ausführungsform für -(C₁-C₆)-Alkyl, -O-(C₁-C₆)-Alkyl, Aryl oder Hetaryl ausgewählt aus der Gruppe Thienyl, Pyrrolyl, Imidazolyl, Pyrazolyl, Pyridyl, Pyrimidinyl, Pyrazinyl oder Pyridazinyl, in einer anderen Ausführungsform für -(C₁-C₆)-Alkyl, Aryl oder Hetaryl ausgewählt aus der Gruppe Thienyl, Pyrrolyl, Imidazolyl, Pyrazolyl, Pyridyl, Pyrimidinyl, Pyrazinyl oder Pyridazinyl, in einer anderen Ausführungsform für Aryl oder Hetaryl ausgewählt aus der Gruppe Thienyl, Pyrrolyl, Imidazolyl, Pyrazolyl, Pyridyl, Pyrimidinyl, Pyrazinyl oder Pyridazinyl, in einer anderen Ausführungsform für Phenyl, wobei das für die andere der Gruppen R1 und R2 stehende Alkyl, -O-Alkyl, Aryl, Phenyl oder Hetaryl in einer Ausführungsform jeweils unsubstituiert oder einfach, zweifach oder dreifach unabhängig voneinander durch F, Cl, Br, CN, -(C₁-C₄)-Alkyl, -O-(C₁-C₄)-Alkyl, oder-CF₃ substituiert ist, in einer anderen Ausführungsform unsubstituiert oder einfach, zweifach oder dreifach unabhängig voneinander durch F, Cl, Br, -(C₁-C₄)-Alkyl, -O-(C₁-C₄)-Alkyloder -CF₃ substituiert ist, in einer anderen Ausführungsform unsubstituiert oder einfach, zweifach oder dreifach unabhängig voneinander durch F, Cl, Br, -(C₁-C₄)-Alkyl oder -CF₃ substituiert ist, in einer anderen Ausführungsform unsubstituiert oder einfach, zweifach oder dreifach unabhängig voneinander durch F, Cl, Br oder -(C₁-C₄)-Alkyl substituiert ist. In einer Ausführungsform der Erfindung sind substituierte Gruppen, die für R1 oder R2 stehen, durch einen oder zwei der angegebenen Substituenten substituiert, die gleich oder verschieden sein können.

In einer Ausführungsform der Erfindung steht R1 in den Verbindungen der Formel la für Phenyl oder Hetaryl, wobei Hetaryl für eine oder mehrere der spezifischen Bedeutungen von Hetaryl in der Definition der Verbindungen der Formel la steht, in einer anderen Ausführungsform steht R1 für Phenyl, wobei Phenyl und Hetaryl in einer Ausführungsform jeweils unsubstituiert oder einfach oder zweifach unabhängig voneinander substituiert sind durch F, Cl, Br, CN, (C₁-C₄)-Alkyl, -O-(C₁-C₄)-Alkyl oder -CF₃, in einer anderen Ausführungsform jeweils unsubstituiert oder einfach oder zweifach unabhängig voneinander substituiert sind durch F, Cl, Br, (C₁-C₄)-Alkyl oder -CF₃, und in einer anderen Ausführungsform jeweils unsubstituiert oder einfach oder zweifach unabhängig voneinander substituiert sind durch F, Cl, Br, oder (C₁-C₄)-Alkyl.

In einer Ausführungsform der Erfindung steht R3 in den Verbindungen der Formel I für -OH, -O-(C₁-C₈)-Alkyl, -O-(C₁-C₆)-Alkylen-NH-C(O)-O-(C₁-C₄)-Alkyl, -O-(C₁-C₆)-Alkylen-NH₂, -NH₂, -NH-(C₁-C₈)-Alkyl, -NH-(C₁-C₆)-Alkylen-Aryl, -NH-(C₁-C₈)-Alkylen-O-(C₁-C₄)-Alkyl, -NH-(C₁-C₈)-Alkyl-OH, -NH-(C₁-C₆)-Alkylen-NH-C(O)-O-(C₁-C₄)-Alkyl, -NH-(C₁-C₆)-Alkylen-NH₂, -NH-(C₀-C₆)-Alkylen-(C₃-C₇)-Cycloalkyl, -NH-(C₀-C₆)-Alkylen-Hetaryl oder -N((C₁-C₄)-Alkyl)₂, in einer anderen Ausführungsform für -OH, -O-(C₁-C₈)-Alkyl, -NH₂, -NH-(C₁-C₈)-Alkyl, -NH-(C₁-C₆)-Alkylen-Aryl, -NH-(C₁-C₈)-Alkylen-O-(C₁-C₄)-Alkyl, -NH-(C₁-C₈)-Alkyl-OH, -NH-(C₁-C₆)-Alkylen-NH-C(O)-O-(C₁-C₄)-Alkyl, -NH-(C₁-C₆)-Alkylen-NH₂, -NH-(C₀-C₆)-Alkylen-(C₃-C₇)-Cycloalkyl oder -NH-(C₀-C₆)-Alkylen-Hetaryl, in einer anderen Ausführungsform für -NH₂, -NH-(C₁-C₈)-Alkyl, -NH-(C₁-C₆)-Alkylen-Aryl, -NH-(C₁-C₈)-Alkylen-O-(C₁-C₄)-Alkyl, -NH-(C₁-C₈)-Alkyl-OH, -NH-(C₁-C₆)-Alkylen-NH-C(O)-O-(C₁-C₄)-Alkyl, -NH-(C₁-C₆)-Alkylen-NH₂, -NH-(C₀-C₆)-Alkylen-(C₃-C₇)-Cycloalkyl oder -NH-(C₀-C₆)-Alkylen-Hetaryl, in einer anderen Ausführungsform für -NH-(C₁-C₈)-Alkyl, -NH-(C₁-C₆)-Alkylen-Aryl, -NH-(C₁-C₈)-Alkylen-O-(C₁-C₄)-Alkyl, -NH-(C₁-C₈)-Alkyl-OH, -NH-(C₁-C₆)-Alkylen-NH-C(O)-O-(C₁-C₄)-Alkyl, -NH-(C₁-C₆)-Alkylen-NH₂, -NH-(C₀-C₆)-Alkylen-(C₃-C₇)-Cycloalkyl oder -NH-(C₀-C₆)-Alkylen-Hetaryl, wobei Aryl, Cycloalkyl und Hetaryl jeweils unsubstituiert oder einfach, zweifach oder dreifach unabhängig voneinander durch F, Cl, -OH, -(C₁-C₄)-Alkyl, -(C₁-C₄)-Alkylen-NH₂, -(C₁-C₄)-Alkylen-OH oder -O-(C₁-C₄)-Alkyl substituiert sind. In einer Ausführungsform steht Hetaryl, das in R3 in Verbindungen der Formel I enthalten ist, für eine oder mehrere der spezifischen Bedeutungen, die Hetaryl in den Verbindungen der Formel I haben kann.

In einer Ausführungsform steht R3 in den Verbindungen der Formel la für -OH, -O-(C₁-C₆)-Alkyl, -NH-(C₁-C₄)-Alkyl, -NH-Benzyl oder -NH-Methylen-(C₃-C₆)-Cycloalkyl, in einer anderen Ausführungsform für -NH-(C₁-C₄)-Alkyl, -NH-Benzyl oder -NH-Methylen-(C₃-C₆)-Cycloalkyl, wobei Cycloalkyl unsubstituiert oder einfach durch -(C₁-C₄)-Alkyl, -(C₁-C₄)-Alkylen-NH₂ oder -CH₂-OH substituiert ist.

In einer Ausführungsform der Erfindung sind R4 und R5 in den Verbindungen der Formel I gleich oder verschieden und stehen unabhängig voneinander für Wasserstoff oder -(C₁-C₆)-Alkyl, oder R4 und R5 bilden zusammen mit dem C-Atom, an das sie gebunden sind, (C₃-C₇)-Cycloalkyl, d. h. einen 3-gliedrigen bis 7-gliedrigen Cycloalkanring, der mit dem die Gruppe X enthaltenden Ring spirocyclisch verknüpft ist. In einer anderen Ausführungsform sind R4 und R5 gleich oder verschieden und stehen unabhängig voneinander für Wasserstoff oder -(C₁-C₆)-Alkyl, oder R4 und R5 bilden zusammen mit dem C-Atom, an das sie gebunden sind, (C₃-C₆)-Cycloalkyl. In einer anderen Ausführungsform sind R4 und R5 gleich oder verschieden und stehen unabhängig voneinander für Wasserstoff oder -(C₁-C₄)-Alkyl, oder R4 und R5 bilden zusammen mit dem C-Atom, an das sie gebunden sind, Cyclobutyl oder Cyclopentyl, d. h. einen Cyclobutanring oder Cyclopentanring, der mit dem die Gruppe X enthaltenden Ring spirocyclisch verknüpft ist. In einer Ausführungsform der Erfindung sind R4 und R5 in den Verbindungen der Formel I gleich oder verschieden und stehen unabhängig voneinander für -(C₁-C₆)-Alkyl, oder R4 und R5 bilden zusammen mit dem C-Atom, an das sie gebunden sind, (C₃-C₇)-Cycloalkyl. In einer anderen Ausführungsform sind R4 und R5 gleich oder verschieden und stehen unabhängig voneinander für -(C₁-C₆)-Alkyl, oder R4 und R5 bilden zusammen mit dem C-Atom, an das sie gebunden sind, (C₃-C₆)-Cycloalkyl. In einer anderen Ausführungsform sind R4 und R5 gleich oder verschieden und stehen unabhängig voneinander für -(C₁-C₄)-Alkyl, oder R4 und R5 bilden zusammen mit dem C-Atom, an das sie gebunden sind, Cyclobutyl oder Cyclopentyl. In einer Ausführungsform der Erfindung bilden R4 und R5 in den Verbindungen der Formel I zusammen mit dem C-Atom, an das sie gebunden sind, (C₃-C₇)-Cycloalkyl, in einer anderen Ausführungsform (C₃-C₆)-Cycloalkyl, in einer anderen Ausführungsform Cyclobutyl oder Cyclopentyl, in einer anderen Ausführungsform Cyclopentyl.

In einer Ausführungsform der Erfindung sind R4 und R5 in den Verbindungen der Formel la gleich oder verschieden und stehen unabhängig voneinander für Wasserstoff oder -(C₁-C₄)-Alkyl, oder R4 und R5 bilden zusammen mit dem C-Atom, an das sie gebunden sind, Cyclobutyl, Cyclopentyl oder Cyclohexyl, d. h. einen Cyclobutanring, Cyclopentanring oder Cyclohexanring, der mit dem die Gruppe X enthaltenden Ring spirocyclisch verknüpft ist. In einer anderen Ausführungsform sind R4 und R5 gleich oder verschieden und stehen unabhängig voneinander für Wasserstoff oder -(C₁-C₄)-Alkyl, oder R4 und R5 bilden zusammen mit dem C-Atom, an das sie gebunden sind, Cyclobutyl oder Cyclopentyl. In einer Ausführungsform der Erfindung sind R4 und R5 in den Verbindungen der Formel la gleich oder verschieden und stehen unabhängig voneinander für -(C₁-C₄)-Alkyl, oder R4 und R5 bilden zusammen mit dem C-Atom, an das sie gebunden sind, Cyclobutyl, Cyclopentyl oder Cyclohexyl. In einer anderen Ausführungsform sind R4 und R5 gleich oder verschieden und stehen unabhängig voneinander für -(C₁-C₄)-Alkyl, oder R4 und R5 bilden zusammen mit dem C-Atom, an das sie gebunden sind, Cyclobutyl oder Cyclopentyl. In einer Ausführungsform der Erfindung bilden R4 und R5 in den Verbindungen der Formel la zusammen mit dem C-Atom, an das sie gebunden sind, Cyclopropyl, Cyclobutyl, Cyclopentyl oder Cyclohexyl, in einer anderen Ausführungsform Cyclobutyl, Cyclopentyl oder Cyclohexyl, in einer anderen Ausführungsform Cyclobutyl oder Cyclopentyl, in einer anderen Ausführungsform Cyclopentyl.

In einer Ausführungsform der Erfindung steht X in den Verbindungen der Formel I für eine kovalente Bindung, CH₂, CH((C₁-C₆)-Alkyl) oder C((C₁-C₄)-Alkyl)₂, in einer anderen Ausführungsform für CH₂, CH((C₁-C₆)-Alkyl) oder C((C₁-C₄)-Alkyl)₂, in einer anderen Ausführungsform für CH₂ oder C((C₁-C₄)-Alkyl)₂, in einer anderen Ausführungsform für CH₂, in einer anderen Ausführungsform für C((C₁-C₄)-Alkyl)₂.

In einer Ausführungsform der Erfindung steht X in den Verbindungen der Formel la für eine kovalente Bindung, CH₂ oder C((C₁-C₄)-Alkyl)₂, in einer anderen Ausführungsform für CH₂ oder C((C₁-C₄)-Alkyl)₂, in einer anderen Ausführungsform für CH₂, in einer anderen Ausführungsform für C((C₁-C₄)-Alkyl)₂.

In einer Ausführungsform der Erfindung steht R6 in den Verbindungen der Formel I für Wasserstoff oder -(C₁-C₆)-Alkyl, in einer anderen Ausführungsform für Wasserstoff, in einer anderen Ausführungsform für -(C₁-C₆)-Alkyl.

In einer Ausführungsform der Erfindung steht R7 in den Verbindungen der Formel I für Wasserstoff oder -(C₁-C₆)-Alkyl, in einer anderen Ausführungsform für Wasserstoff, in einer anderen Ausführungsform für -(C₁-C₆)-Alkyl.

Die Erfindung betrifft alle Kombinationen von Definitionen der Verbindungen der Formeln I und la und einer oder mehrerer der beschriebenen jeweiligen Ausführungsformen, wie auch alle Kombinationen von Definitionen der Verbindungen der Formeln I und la und einer oder mehrerer der beschriebenen jeweiligen Ausführungsformen und/oder einer oder mehrerer der beschriebenen spezifischen Bedeutungen, die eine Gruppe in den Verbindungen der Formeln I und la haben kann.

Die erfindungsgemäßen Verbindungen können nach wohlbekannten Verfahren oder nach hier beschriebenen Verfahren hergestellt werden. Die Erfindung betrifft auch ein Verfahren zur Herstellung einer Verbindung der Formel I oder la und/oder einer stereoisomeren oder tautomeren Form der Verbindung der Formel I oder la und/oder eines physiologisch verträglichen Salzes der Verbindung der Formel I oder la, nach dem die Verbindungen der Formel I und la, ihre stereoisomeren oder tautomeren Formen oder ihre physiologisch verträglichen Salze hergestellt werden können und das dadurch gekennzeichnet ist, dass man
a) eine Verbindung der Formel II, 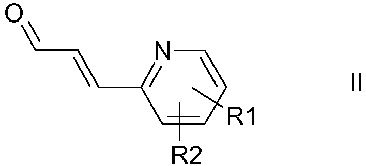 wobei die Reste R1 und R2 wie in Formel I oder la definiert sind, mit einer Verbindung der Formel III und einer Verbindung der Formel IV, 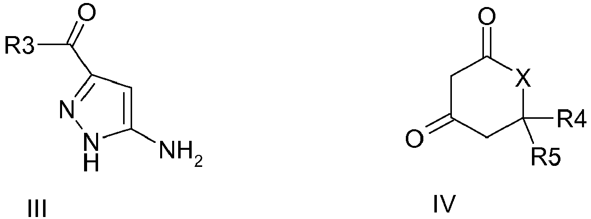 wobei die Reste R1 und R2 wie in Formel I oder la definiert sind, in Gegenwart eines geeigneten Lösungsmittels oder Lösungsmittelgemisches bei 20°C bis 120°C zu einer Verbindung der Formel I oder la umsetzt; oder
b) eine Verbindung der Formel V, 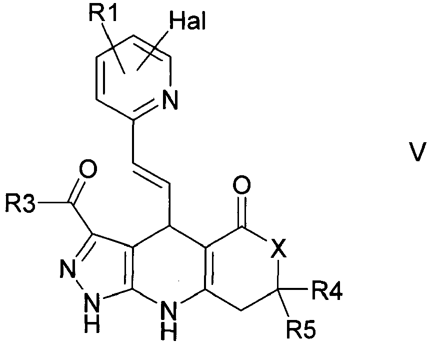 wobei die Reste X, R1, R3, R4 und R5 wie in Formel I oder la definiert sind und HaI die Bedeutung Chlor, Brom, Iod oder Triflat (Trifluormethansulfonyloxy) hat, mit einer Verbindung der Formel R2-B(OH)₂ oder einem Derivat davon in Gegenwart einer Base und eines geeigneten Metallkatalysators in einem geeigneten Lösemittel oder Lösemittelgemisch zu einer Verbindung der Formel I oder la umsetzt; oder
c) eine Verbindung der Formel I, worin X die Bedeutung NH hat, mit einem geeigneten Alkylierungsmittel in Gegenwart einer Base in einem geeigneten inerten Lösemittel oder Lösungmittelgemisch bei Raumtemperatur oder bei erhöhter Temperatur zu einer Verbindung der Formel I umsetzt, worin X die Bedeutung N-R6 hat und R6 -(C₁-C₆)-Alkyl, -(C₁-C₆)-Alkylen-(C₃-C₇)-Cycloalkyl oder -(C₁-C₆)-Alkylen-Aryl bedeutet; oder
d) eine Verbindung der Formel I oder la, worin R3 für -OH, -O-Aryl oder -O-(C₁-C₆)-Alkyl steht und R1, R2, R4, R5 und X wie in Formel I oder la definiert sind, nach gängigen Verfahren zu einer Verbindung der Formel I oder la umsetzt, worin R3 für -NH₂, -NH-(C₁-C₆)-Alkyl, -NH-(C₁-C₆)-Alkylen-Aryl, -NH-(C₁-C₆)-Alkylen-(C₃-C₇)-Cycloalkyl, -NH-(C₁-C₆)-Alkylen-Hetaryl oder -N((C₁-C₄)-Alkyl)₂, wobei Aryl und Hetaryl jeweils unsubstituiert oder einfach,
   zweifach oder dreifach unabhängig voneinander durch F, Cl, -OH, -(C₁-C₄)-Alkyl, -O-(C₁-C₄)-Alkyl, Aryl oder Hetaryl substituiert sind; oder
e) die nach den Verfahren a) bis d) hergestellte Verbindung der Formel I oder la, oder eine geeignete Vorstufe der Verbindung der Formel I oder la, die aufgrund ihrer chemischen Struktur in enantiomeren oder diastereomeren Formen auftritt, durch Salzbildung mit enantiomerenreinen Säuren oder Basen, Chromatographie an chiralen Stationärphasen oder Derivatisierung mittels chiraler enantiomerenreiner Verbindungen wie zum Beispiel Aminosäuren, Trennung der somit erhaltenen Diastereomeren, und Abspaltung der chiralen Hilfsgruppen in die reinen Enantiomeren oder Diastereomeren auftrennt; oder
f) die nach den Verfahren a) bis e) hergestellte Verbindung der Formel I oder la entweder in freier Form isoliert, oder aus einem physiologisch unverträglichen Salz freisetzt, oder im Falle des Vorliegens von sauren oder basischen Gruppen in ein physiologisch verträgliches Salz umwandelt.

Die Verbindungen der Formel II können hergestellt werden durch Umsetzung eines Aldehyds der Formel VI mit einer Verbindung der Formel VII 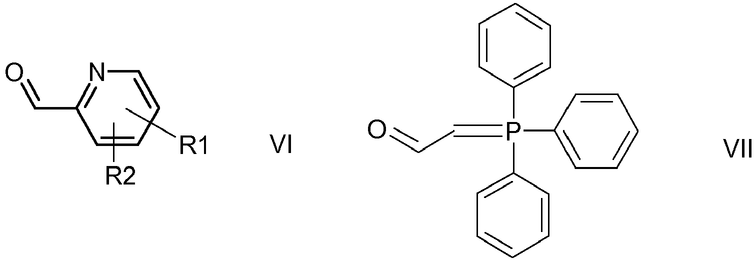 oder anderen geeigneten Phosphorylid-Reagenzien in einem geeigneten Lösemittel und gegebenenfalls bei erhöhter Temperatur. Aldehyde der Formel VI sind entweder kommerziell erhältlich oder lassen sich nach bekannten Verfahren herstellen. So kann man beispielsweise halogensubstituierte Pyridin-2-aldehyde in Gegenwart von geeigneten Übergangsmetallkatalysatoren wie Palladium oder Nickel und deren Phosphankomplexen mit Alkyl-, Aryl- und Hetaryl-Borsäurederivaten oder entsprechenden Borsäureesterderivaten umsetzen zu Alkyl-, Aryl- und Hetarylsubstituierten Derivaten der Formel VI.

Saure oder basische Verbindungen der Formel I oder la können in Form ihrer Salze oder in freier Form vorliegen. Bevorzugt sind pharmakologisch verträgliche Salze, insbesondere pharmazeutisch akzeptable Salze, beispielsweise Alkali- oder Erdalkalimetallsalze oder Hydrochloride, Sulfate, Hemisulfate, Methylsulfonate, p-Toluolsulfonate, Phosphate sowie Salze der Aminosäuren, natürlicher Basen oder Carbonsäuren wie Lactate, Citrate, Tartrate, Acetate, Adipinate, Fumarate, Gluconate, Glutamate, Maleinate oder Palmoate. Die Herstellung physiologisch verträglicher Salze aus zur Salzbildung befähigten Verbindungen der Formel I, einschließlich deren stereoisomeren Formen, gemäß Verfahrensschritt f) erfolgt in an sich bekannter Weise. Enthalten Verbindungen der Formel I oder la saure Funktionalität, so lassen sich mit basischen Reagenzien wie Hydroxiden, Carbonaten, Hydrogencarbonaten, Alkoholaten sowie Ammoniak oder organischen Basen, beispielsweise Trimethylamin oder Triethylamin, Ethanolamin, Diethanolamin oder Triethanolamin, Trometamol oder auch basischen Aminosäuren, etwa Lysin, Ornithin oder Arginin, stabile Alkali-, Erdalkali- oder gegebenenfalls substituierte Ammoniumsalze bilden. Basische Gruppen der Verbindungen der Formel I, bilden mit Säuren Säureadditionssalze. Hierfür kommen sowohl anorganische als auch organische Säuren wie Chlorwasserstoff-, Bromwasserstoff-, Schwefel-, Hemischwefel-, Phosphor-, Methansulfon-, Benzolsulfon-, p-Toluolsulfon-, 4-Brombenzolsulfon-, Cyclohexylamidosulfon-, Trifluormethylsulfon-, 2-Hydroxyethansulfon-, Essig-, Oxal-, Wein-, Bernstein-, Glycerolphosphor-, Milch-, Äpfel-, Adipin-, Citronen-, Fumar-, Malein-, Glucon-, Glucuron-, Palmitin- oder Trifluoressigsäure in Frage.

Die Verbindungen der Formel I und la können ein oder mehrere asymmetrische Kohlenstoffatome enthalten und in Form von Diastereomeren oder Enantiomeren oder Gemischen derselben auftreten. Sofern eine Verbindung der Formel I oder la als Gemisch von Diastereomeren oder Enantiomeren auftritt oder bei der gewählten Synthese als deren Gemisch anfällt, kann sie in die reinen Stereoisomeren getrennt werden, entweder durch Chromatographie an einem gegebenenfalls chiralen Trägermaterial, oder, sofern die racemische Verbindung der Formel I oder la zur Salzbildung befähigt ist, kann auch eine fraktionierte Kristallisation der mit einer optisch aktiven Base oder Säure als Hilfsstoff gebildeten diastereomeren Salze durchgeführt werden. Als chirale Stationärphasen für die dünnschichtchromatographische oder säulenchromatographische Trennung von Enantiomeren eignen sich zum Beispiel modifizierte Kieselgelträger (sogenannte Pirkle-Phasen) sowie hochmolekulare Kohlenhydrate wie Triacetylcellulose. Für analytische Zwecke sind nach entsprechender, dem Fachmann bekannter Derivatisierung, auch gaschromatographische Methoden an chiralen Stationärphasen anwendbar. Zur Enantiomerentrennung der racemischen Carbonsäuren werden mit einer optisch aktiven, in der Regel kommerziell erhältlichen Base wie (-)-Nicotin, (+)- und (-)-Phenylethylamin, Chininbasen, L-Lysin oder L- und D-Arginin die unterschiedlich löslichen diastereomeren Salze gebildet, die schwerer lösliche Komponente als Feststoff isoliert, das leichter lösliche Diastereomer aus der Mutterlauge abgeschieden und aus den so gewonnenen diastereomeren Salzen die reinen Enantiomeren gewonnen. Auf prinzipiell gleiche Weise kann man die racemischen Verbindungen der Formel I oder la, die eine basische Gruppe wie eine Aminogruppe enthalten, mit optisch aktiven Säuren, wie (+)-Campher-10-sulfonsäure, D- und L-Weinsäure, D- und L- Milchsäure sowie (+)-und (-)-Mandelsäure in die reinen Enantiomeren überführen. Auch kann man chirale Verbindungen, die Alkohol- oder Amin-Funktionen enthalten, mit entsprechend aktivierten oder gegebenenfalls N-geschützten enantiomerenreinen Aminosäuren in die entsprechenden Ester oder Amide überführen, oder umgekehrt chirale Carbonsäuren mit carboxygeschützten enantiomerenreinen Aminosäuren in die Amide oder mit enantiomerenreinen Hydroxycarbonsäuren wie Milchsäure, in die entsprechenden chiralen Ester überführen. Sodann kann die Chiralität des in enantiomerenreiner Form eingebrachten Aminosäurerestes oder Alkoholrestes zur Trennung der Isomeren genutzt werden, indem man eine Trennung der nunmehr vorliegenden Diastereomeren durch Kristallisation oder Chromatographie an geeigneten Stationärphasen vornimmt und danach den mitgeführten chiralen Molekülteil mittels geeigneter Methoden wieder abspaltet.

Weiterhin ergibt sich bei einigen der Verbindungen der Formel I oder la die Möglichkeit zur Herstellung der Gerüststrukturen aus diastereomerenreinen oder enantiomerenreinen Ausgangsprodukten. Dadurch können auch andere oder vereinfachte Verfahren zur Aufreinigung der Endprodukte eingesetzt werden. Diese Ausgangsprodukte wurden zuvor nach literaturbekannten Verfahren enantiomerenrein oder diastereomerenrein hergestellt. Das kann insbesondere bedeuten, dass in der Synthese der Grundgerüste entweder enantioselektive Verfahren zum Einsatz kommen, oder aber eine Enantiomerentrennung oder Diastereomerentrennung auf früher Synthesestufe und nicht erst auf der Stufe der Endprodukte durchgeführt wird. Ebenso kann eine Vereinfachung der Trennungen dadurch erreicht werden, dass zweistufig oder mehrstufig vorgegangen wird.

Die Erfindung betrifft auch Arzneimittel und pharmazeutische Zusammensetzungen, gekennzeichnet durch einen wirksamen Gehalt an mindestens einer Verbindung der Formel I oder la und/oder eines physiologisch verträglichen Salzes der Verbindung der Formel I oder la und/oder einer stereoisomeren oder tautomeren Form der Verbindung der Formel I oder la, zusammen mit einem pharmazeutisch geeigneten und physiologisch verträglichen Trägerstoff, Zusatzstoff und/oder anderen Wirkstoffen und Hilfsstoffen. Die Erfindung betrifft weiterhin eine Verbindung der Formel I oder la und/oder alle stereoisomeren oder tautomeren Formen der Verbindung der Formel I oder la und/oder Gemische dieser Formen in jedem Verhältnis, und/oder ein physiologisch verträgliches Salz der Verbindung der Formel I oder la, zur Verwendung als Pharmazeutikum oder Wirkstoff in einem Arzneimittel.

Aufgrund der pharmakologischen Eigenschaften eignen sich die erfindungsgemäßen Verbindungen beispielsweise zur Prophylaxe, Sekundär-Prävention und Therapie all solcher Erkrankungen, die durch eine Hemmung des Protease-aktivierten Rezeptors 1 (PAR1) behandelbar sind. So eignen sich die erfindungsgemäßen Verbindungen sowohl für eine prophylaktische als auch für eine therapeutische Anwendung am Menschen. Sie eignen sich sowohl für eine akute Behandlung als auch für eine chronische Behandlung in einer Langzeittherapie. Die Verbindungen der Formeln I und la können eingesetzt werden in Patienten, die an Störungen des Wohlbefindens oder Krankheiten leiden, die mit Thrombosen, Embolien, Hyperkoagulabilität oder fibrotischen Veränderungen einhergehen. Dazu gehören der Myokardinfarkt, die Angina pectoris und alle andere Formen des akuten Koronarsyndroms, den akuten Schlaganfall oder dessen Sekundärprevention, die peripher vaskulären Erkrankungen, die tiefe Venenthrombose, die Lungenembolie, embolische oder thrombotische Ereignisse bedingt durch kardiale Arrhythmien, kardiovaskuläre Ereignisse wie Restenose nach Revaskularisierung, Angioplastie und ähnlichen Eingriffen wie Stentimplantationen und Bypass-Operationen.

Weiterhin können die Verbindungen der Formeln I und la eingesetzt werden bei allen Eingriffen, die zu einem Kontakt des Blutes mit Fremdoberflächen führen wie bei Dialysepatienten und Patienten mit Verweilkathetern. Verbindungen der Formeln I und la können eingesetzt werden, um die Thrombosegefahr nach chirurgischen Eingriffen wie bei Knie- und Hüftgelenksoperationen zu reduzieren. Verbindungen der Formeln I und la eignen für die Behandlung von Patienten mit disseminierter intravaskulärer Koagulation, Sepsis und anderen intravaskulären Ereignissen, die mit einer Entzündung einhergehen.

Weiterhin eignen sich Verbindungen der Formeln I und la für die Prophylaxe und Behandlung von Patienten mit Atherosklerose, Diabetes und dem metabolischen Syndrom und deren Folgen. Störungen des hämostatischen Systems (beispielsweise Fibrinablagerungen) wurden impliziert in Mechanismen, die zu Tumorwachstum und Tumormetastasierung führen, sowie bei entzündlichen und degenerativen Gelenkserkrankungen wie der rheumatoiden Arthritis und der Arthrose. Verbindungen der Formeln I und la eignen sich zur Verlangsamung oder Verhinderung solcher Prozesse.

Weitere Indikationen für den Einsatz der Verbindungen der Formeln I und la sind fibrotische Veränderungen der Lunge wie die chronische obstruktive Lungenerkrankung, das adult respiratory distress syndrome (ARDS) und des Auges wie Fibrinablagerungen nach Augenoperationen. Verbindungen der Formeln I und la eignen sich auch zur Verhinderung und/oder Behandlung von Narbenbildung.

Die Applikation der erfindungsgemäßen Arzneimittel kann zum Beispiel durch orale, inhalative, rektale oder transdermale Applikation oder durch subkutane, intraartikuläre, intraperitoneale oder intravenöse Injektion erfolgen. Bevorzugt ist die orale Applikation. Auch eine Beschichtung von Stents mit Verbindungen der Formeln I und la und anderen Oberflächen, die im Körper mit Blut in Kontakt kommen, ist möglich.

Die Erfindung betrifft auch ein Verfahren zur Herstellung eines Arzneimittels, das dadurch gekennzeichnet ist, dass man mindestens eine Verbindung der Formel I oder la mit einem pharmazeutisch geeigneten und physiologisch verträglichen Träger und gegebenenfalls weiteren geeigneten Wirkstoffen, Zusatzstoffen oder Hilfsstoffen in eine geeignete Darreichungsform bringt.

Geeignete feste oder galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen und injizierbare Lösungen sowie Präparate mit protrahierter Wirkstoff-Freigabe, bei deren Herstellung übliche Hilfsmittel wie Trägerstoffe, Sprengmittel, Bindemittel, Überzugsmittel, Quellungsmittel, Gleitmittel oder Schmiermittel, Geschmacksstoffe, Süßungsmittel und Lösungsvermittler Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumenöl, Erdnussöl oder Sesamöl, Polyethylenglykol und Lösungsmittel, wie etwa steriles Wasser und einwertige oder mehrwertige Alkohole wie Glycerin, genannt.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel I oder la enthält. Bei festen Dosierungseinheiten wie Tabletten, Kapseln, Dragees oder Suppositorien, kann diese Dosis bis zu etwa 1000 mg, bevorzugt etwa 50 mg bis 300 mg, und bei Injektionslösungen in Ampullenform bis zu etwa 300 mg, bevorzugt etwa 10 mg bis 100 mg, betragen.

Für die Behandlung eines erwachsenen, etwa 70 kg schweren Patienten sind je nach Wirksamkeit der Verbindung der Formel I oder la, Tagesdosen von etwa 2 mg bis 1000 mg Wirkstoff, bevorzugt etwa 50 mg bis 500 mg, indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Verbindungen der Formel I oder la können sowohl als Monotherapie als auch in Kombination oder gemeinsam mit allen Antithrombotika (Antikoagulanzien und Plättchenaggregationshemmer), Thrombolytika (Plasminogenaktivatoren jeglicher Art), anderen profibrinolytisch wirksamen Substanzen, Blutdrucksenkern, Regulatoren des Blutzuckers, Lipidsenkern und Antiarrhythmika verabreicht werden. Als Plättchenaggregationshemmer kommen dabei Cyclooxygenase 1-Inhibitoren wie Aspirin, irreversible P2Y₁₂-Antagonisten wie Clopidogrel oder Prasugrel, reversible P2Y₁₂₋Antagonisten wie Cangrelor oder AZD6140 und Thromboxan A₂/Prostaglandin H₂-Antagonisten wie Terutroban in Frage. Additive Effekte der PAR1-Blockade in Kombination mit P2Y₁₂-Blockade konnten beispielsweise gezeigt werden (M. Chintala et al., Eur. Heart J. 28 (Abstract Supplement 1): 188, 2007).

### Beispiele

Die hergestellten Verbindungen wurden in der Regel durch spektroskopische und chromatographische Daten, im speziellen Massenspektren (MS) und HPLC-Retentionszeiten (Rt; in min), charakterisiert, welche durch kombinierte, analytische HPLC/MS-Charakterisierung (LC/MS) erhalten wurden. In der MS-Charakterisierung ist in der Regel die Massennummer (m/z) des Peaks des Molekülions (M bzw. M⁺) oder eines verwandten Ions wie des Ions M+1 (bzw. M+1⁺; protoniertes Molekülion M+H⁺), welches sich abhängig von der Ionisierungsmethode, die benutzt wurde, gebildet hat, angegeben. Im Allgemeinen wurde die Ionisationsmethode Elektrospray-Ionisation (ESI) benutzt. Folgende LC/MS-Methoden wurden benutzt:

### Methode B

Säule: Chirapak IA/103 250x4,6 mm
Lösungsmittel: Hep:EtOH:MeOH, isokratisch 10:1:1
Ionisation: ESI⁺

### Methode C

Säule: Chirapak IA/103 250x4,6 mm
Lösungsmittel: Hep:EtOH:MeOH, vorkonditioniert mit Diethylamin, isokratisch 10:1:1
Ionisation: ESI⁺

### Methode D

Säule: YMC J'sphere ODS H80 20x2,1 mm 4 µm
Lösungsmittel: MeCN:H₂O + 0.05% TFA (Fluss 1 mL/min)
Gradient: von 4:96 (0 min) bis 95:5 (2 min) bis 95:5 (2,4 min) bis 96:4 (2,45 min)
Ionisation: ESI⁺

### Methode E

Säule: Chirapak IA/104 250x4,6 mm
Lösungsmittel: MeCN + 0,1% Diethylamin, isokratisch
Ionisation: ESI⁺

### Methode J

Säule: Luna C18 10x2 mm 3 µm
Lösungsmittel: MeCN + 0.05% TFA:H₂O + 0.05% TFA (Fluss 1,1 mL/min)
Gradient: 7:93 (0 min) bis 95:5 (1,2 min) bis 95:5 (1,4 min) bis 7:93 (1,45 min)
Ionisation: ESI⁺

Weiterhin erfolgte eine Charakterisierung der Verbindungen durch ¹H-NMR-Spektroskopie. Verwendete Abkürzungen sind entweder erläutert oder entsprechen den üblichen Konventionen. Das Abdampfen von Lösungsmitteln geschah in der Regel unter vermindertem Druck bei 35 °C bis 45 °C am Rotationsverdampfer und wird mit "vom Lösungsmittel befreit", "eingeengt", "abgedampft" oder "Lösungsmittel entfernt" umschrieben. Die Umsetzungen fanden in Standard-Reaktionsapparaturen wie Einhalskolben oder Mehrhalskolben statt, die, wenn nicht anders beschrieben, dem Bedarf angemessen 5 ml bis 2000 ml Volumen fassten und je nach Anforderung mit Septum, Stopfen, Kühler, Rührer oder anderen Ausrüstungsgegenständen bestückt waren. Wenn nicht anders erwähnt, fanden alle Reaktionen unter Argon als Schutzgas statt und wurden mit Magnetrührern gerührt.

### Verwendete Abkürzungen:

- DCM: Dichlormethan
- DME: 1,2-Dimethoxyethan
- DMF: Dimethylformamid
- DMSO: Dimethylsulfoxid
- EDCI: N-(3-Dimethylaminopropyl)-N'-ethylcarbodiimid Hydrochlorid
- EtOH: Ethanol
- ges.: gesättigt
- HATU: O-(7-Azabenzotriazol-1-yl)-N,N,N',N'-tetramethyluronium-hexafluorophosphat
- Hep: Heptan
- HOBt: 1-Hydroxy-1 H-benzotriazol Hydrat
- MeCN: Acetonitril
- MeOH: Methanol
- NMM: N-Methyl-Morpholin
- RT: Raumtemperatur (20 °C bis 25 °C)
- Rt: Retentionszeit
- TFA: Trifluoressigsäure

Bei den Umsetzungen werden in der Regel mehrere Diastereomere gebildet, die sich als racemische Mischungen säulenchromatografisch trennen lassen. Wenn nicht angegeben ist eine Zuordnung dieser racemischen Mischungen zu bestimmten Konfigurationen noch nicht eindeutig erfolgt. Ebenfalls noch nicht erfolgt ist die absolute Zuordnung der reinen Diastereomeren, die aus den racemischen Mischungen durch chirale Säulenchromatografie erhalten werden. Bei Angabe einer definierten Stereochemie erfolgte die Zuordnung aus den Kopplungskonstanten der Wasserstoffatome im Pyrrolring durch NMR-spektroskopische Verfahren.

### Beispiel 1

### 5-(3-Fluor-phenyl)-pyridin-2-carbaldehyd

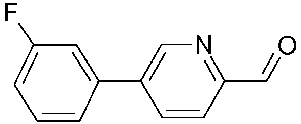

Die Mischung von 2 g 5-Brom-pyridin-2-carbaldehyd, 1,96 g 3-Fluor-phenylboron-säure, 11,2 g K₂CO₃, 160 ml Toluol, 60 ml Wasser, 60 ml Ethanol und 0,93 mg Tetrakis(triphenylphosphin)palladium(0) ist unter Argon bei 100°C 2 Stunden gerührt worden. Die Lösemittel werden abgedampft und der Rückstand in 100 ml Wasser dispergiert und das Produkt wird mit 2 Portionen von 30 ml Ethylacetat extrahiert. Die organische Phase wird mit 30 ml ges. Kochsalzlösung gewaschen, über Natriumsulfat getrocknet und eingeengt. Der resultierende Rückstand wird aus 28 ml Isopropanol umkristallisiert:
Ausbeute: 1,3 g, LC/MS (Methode D): m/z = 202 (M+1); Rt = 1,317 min

### Beispiel 2

### (E)-3-[5-(3-Fluor-phenyl)-pyridin-2-yl]-propenal

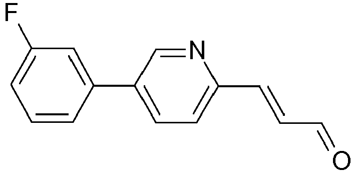

584 mg 5-(3-Fluor-phenyl)-pyridin-2-carbaldehyd und 883 mg (Triphenylphosphor-anyliden)-acetaldehyd werden bei RT über Nacht gerührt. Die Lösemittel werden abgedampft und der Rückstand durch Säulen-Chromatografie (Kieselgel, MeOH:DCM = 99,5:0,5) gereinigt.
Ausbeute: 450 mg, LC/MS (Methode J): m/z = 228 (M+1); Rt = 0,887 min

### Beispiel 3

### (E)-3-(5-Brom-pyridin-2-yl)-propenal

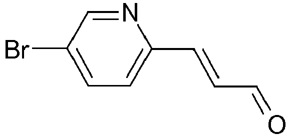

818 mg (Triphenylphosphoranyliden)-acetaldehyd und 500 mg 5-Brom-pyridin-carbaldehyd werden bei RT über Nacht gerührt. Die flüchtigen Anteile werden unter verminderten Druck entfernt und der Rückstand durch Säulenchromatografie (Kieselgel, MeOH:DCM = 99:1) gereinigt.
Ausbeute: 415 mg; LC/MS (Methode D): m/z = 213 (M+1); Rt = 1,121 min

### Beispiel 4

### 5-Nitro-1H-pyrazol-3-carbonsäure-cyclopropylmethylamid

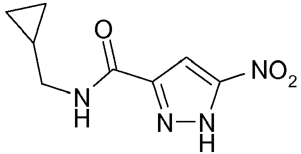

7,5 g 5-Nitro-1H-pyrazol-3-carbonsäure wurden in 70 ml DMF gelöst. 19,9 g HATU und 9,6 g Triethylamin werden bei RT zugefügt und nach 10 Minuten Rühren werden 5,1 g Aminomethylcyclopropan-hydrochlorid zugegeben und die Mischung über Nacht bei RT gerührt. Nach wässriger Aufarbeitung wird das Rohprodukt durch Säulenchromatografie (Kieselgel, Methanol:DCM = 1 : 30) gereinigt.
Ausbeute: 5 g; LC/MS (Methode J): m/z = 211 (M+1); Rt = 0,617 min

### Beispiel 5

### 5-Amino-1 H-pyrazol-3-carbonsäure-cyclopropylmethylamid

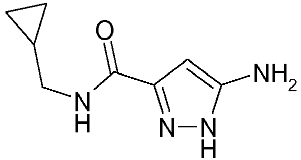

1 g 5-Nitro-1H-pyrazol-3-carbonsäure-cyclopropylmethylamid wird in 30 ml Methanol und 2 ml Essigsäure gelöst. Diese Mischung wird in Gegenwart von 110 mg Pd/C bei 5 bar bis zur vollständigen Umsetzung hydriert. Die Mischung wird über Cellite filtriert, zur Trockene eingedampft und durch Säulenchromatografie (Kieselgel, Methanol:DCM = 1 : 30) gereinigt.
Ausbeute: 805 mg; LC/MS (Methode J): m/z = 181 (M+1); Rt = 0,170 min

Alle 5-Amino-1 H-pyrazol-3-carbonsäureamide wurden auf diese Weise hergestellt, wenn nicht anders angegeben.

### Allgemeine Arbeitsvorschrift zur Dreikomponentenkupplung in Ethanol (Methode A)

Äquimolare Mengen vom entsprechenden Amino-1H-pyrazol-3-carbonsäureamid-Baustein, vom cyclischen Diketon und (E)-3-[5-(3-Fluor-phenyl)-pyridin-2-yl]-propenal wurden in EtOH gelöst und für 2 bis 12 h am Rückfluss erhitzt. Nach dem Erkalten wurde die Mischung eingeengt und durch Säulenchromatografie an Kieselgel gereinigt.

### Allgemeine Arbeitsvorschrift zur Dreikomponentenkupplung in einem 1,4-Dioxan-Wasser-Gemisch (Methode B)

Äquimolare Mengen vom entsprechenden Amino-1H-pyrazol-3-carbonsäureamid-Baustein, vom cyclischen Diketon und (E)-3-[5-(3-Fluor-phenyl)-pyridin-2-yl]-propenal wurden in einem Gemisch aus 1,4-Dioxan/Wasser (2:1) gelöst und für 1 bis 12 h auf 30°C bis 80°C erhitzt. Nach dem Erkalten wurde die Mischung eingeengt und durch Säulenchromatografie an Kieselgel gereinigt.

### Allgemeine Arbeitsvorschrift zur Suzuki-Kupplung (Methode C)

Das entsprechenden Arylbromid (1 Äq), die entsprechende Boronsäure (1,5 Äq) K₃PO₄ (2,5 Äq) und 1,1'-Bis(diphenylphosphino)ferrocen-palladiumdichlorid (20 mol%) wurden in einem Gemisch aus DME/n-Butanol/Wasser (2:2:1) gelöst. Durch die Lösung wurde 10 min Argon geblubbert. Danach wurde in der Mikrowelle 1 bis 4 Stunden auf 120°C bis 150°C erhitzt. Nach dem Erkalten wurde die Mischung eingeengt, der verbleibende Rückstand in DCM aufgenommen und mit ges. NaHCO₃-Lösung gewaschen. Die organische Phase wurde über Na₂SO₄ getrocknet, aufkonzentriert und durch Säulenchromatografie an Kieselgel gereinigt.

### Beispiel 6

### 4-{(E)-2-[5-(3-Fluor-phenyl)-pyridin-2-yl]-vinyl)-7,7-dimethyl-5-oxo-4,5,6,7,8,9-hexahydro-1H-pyrazolo[3,4-b]chinolin-3-carbonsäure-cyclopropylmethylamid

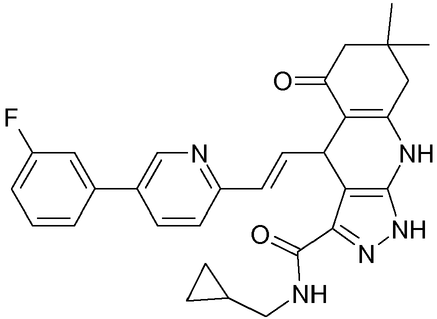

Gemäß der Allgemeinen Vorschrift A wird eine Mischung von 50 mg (E)-3-[5-(3-Fluor-phenyl)-pyridin-2-yl]-propenal, 40 mg 5-Amino-1H-pyrazol-3-carbonsäure-cyclopropylmethylamid, 31 mg Dimedon und 5 ml Ethanol 2 Stunden am Rückfluss gekocht. Nach dem Erkalten wird die Mischung eingeengt und durch Säulenchromatografie (Kieselgel, Methanol:DCM = 5 : 95) gereinigt.
Ausbeute: 25 mg; LC/MS (Methode J): m/z = 512 (M+1); Rt = 0,786 min

### Beispiel 7

### 7,7-Dimethyl-4-[(E)-2-(6-methyl-pyridin-2-yl)-vinyl]-5-oxo-4,5,6,7,8,9-hexahydro-1 H-pyrazolo[3,4-b]chinolin-3-carbonsäure-isopropylamid

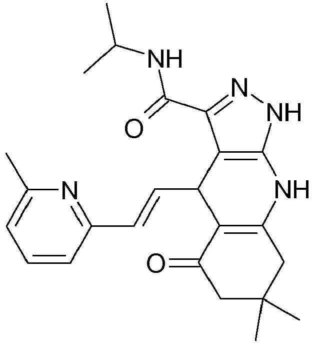

Die Titelverbindung wird analog Arbeitsvorschrift A von 100 mg (E)-3-(6-methyl-pyridin-2-yl)-propenal, 95 mg Dimedon und 114 mg 5-Amino-1H-pyrazol-3-carbonsäure-isopropylamid hergestellt.
Ausbeute: 7 mg; LC/MS (Methode D): m/z = 420 (M+1); Rt = 0,877 min

### Beispiel 8

### 4-{(E)-2-[5-(3-Fluor-phenyl)-pyridin-2-yl]-vinyl)-7,7-dimethyl-5-oxo-4,5,6,7,8,9-hexahydro-1H-pyrazolo[3,4-b]chinolin-3-carbonsäure-isopropylamid

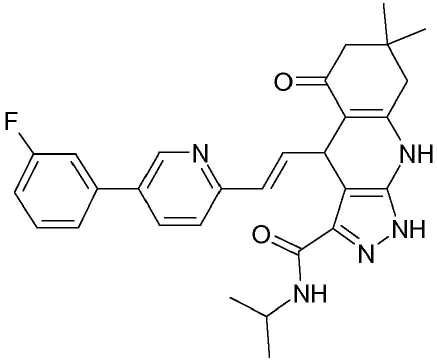

Die Titelverbindung wird gemäß Arbeitsvorschrift A von 50 mg (E)-3-[5-(3-fluor-phenyl)-pyridin-2-yl]-propenal, 31 mg Dimedon und 37 mg 5-Amino-1H-pyrazol-3-carbonsäure-isopropylamid hergestellt.
Ausbeute: 35 mg; LC/MS (Methode J): m/z = 500 (M+1); Rt = 0,766 min

### Beispiel 9

### 4-{(E)-2-[5-(3-Fluor-phenyl)-pyridin-2-yl]-vinyl)-6,6-dimethyl-5-oxo-4,5,6,7,8,9-hexahydro-1H-pyrazolo[3,4-b]chinolin-3-carbonsäure-cyclopropylmethylamid

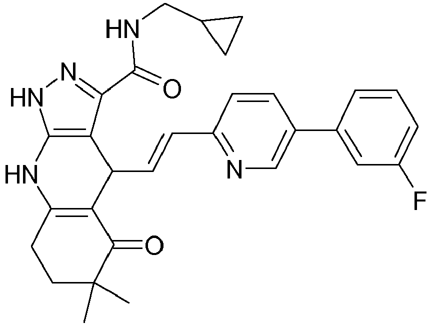

Die Titelverbindung wird gemäß Arbeitsvorschrift A von 50 mg (E)-3-[5-(3-fluor-phenyl)-pyridin-2-yl]-propenal, 31 mg 4,4-Dimethyl-cyclohexan-1,3-dion und 40 mg 5-Amino-1H-pyrazol-3-carbonsäure-cyclopropylmethylamid hergestellt. Ausbeute: 35 mg; LC/MS (Methode J): m/z = 512 (M+1); Rt = 0,784 min

### Beispiel 10

### 4-{(E)-2-[5-(3-Fluor-phenyl)-pyridin-2-yl]-vinyl)-7-isopropyl-5-oxo-4,5,6,7,8,9-hexahydro-1H-pyrazolo[3,4-b]chinolin-3-carbonsäure-cyclopropylmethylamid

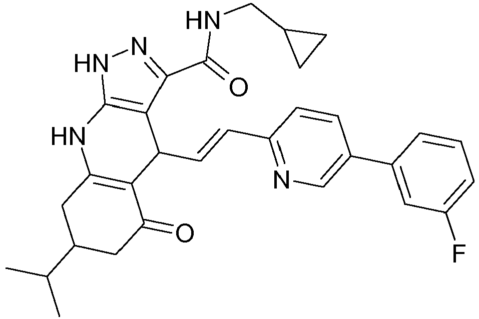

Die Titelverbindung wird als Diastereomerengemisch gemäß Arbeitsvorschrift A von 50 mg (E)-3-[5-(3-fluor-phenyl)-pyridin-2-yl]-propenal, 34 mg 5-Isopropyl-cyclohexan-1,3-dion und 40 mg 5-Amino-1H-pyrazol-3-carbonsäure-cyclopropylmethylamid hergestellt.
Ausbeute: 30 mg; LC/MS (Methode J): m/z = 526 (M+1); Rt = 0,823 min

### Beispiel 11

### 4-{(E)-2-[5-(3-Fluor-phenyl)-pyridin-2-yl]-vinyl)-7-methyl-5-oxo-4,5,6,7,8,9-hexahydro-1 H-pyrazolo[3,4-b]chinolin-3-carbonsäure-isopropylamid

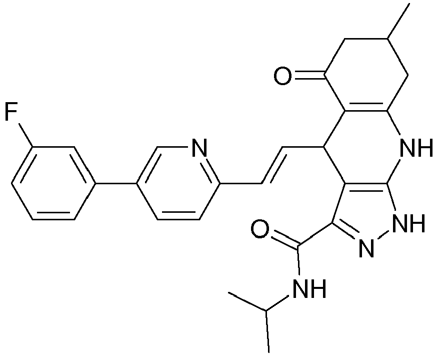

Die Titelverbindung wird als Diastereomerengemisch gemäß Arbeitsvorschrift A von 50 mg (E)-3-[5-(3-fluor-phenyl)-pyridin-2-yl]-propenal, 27 mg 5-Methyl-cyclohexan-1,3-dion und 37 mg 5-Amino-1H-pyrazol-3-carbonsäure-isopropylamid hergestellt. Ausbeute: 35 mg; LC/MS (Methode J): m/z = 486 (M+1); Rt = 0,722 min

### Beispiel 12

### 4-{(E)-2-[5-(3-Fluor-phenyl)-pyridin-2-yl]-vinyl}-7-methyl-5-oxo-4,5,6,7,8,9-hexahydro-1 H-pyrazolo[3,4-b]chinolin-3-carbonsäure-cyclopropylmethylamid

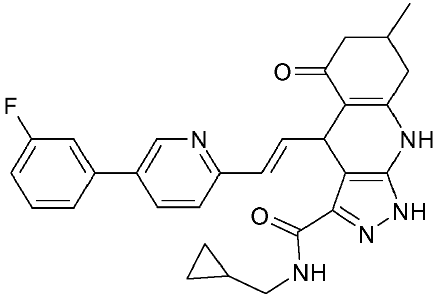

Die Titelverbindung wird als Diastereomerengemisch gemäß Arbeitsvorschrift A von 200 mg (E)-3-[5-(3-fluor-phenyl)-pyridin-2-yl]-propenal, 111 mg 5-Methyl-cyclohexan-1,3-dion und 159 mg 5-Amino-1H-pyrazol-3-carbonsäure-cyclopropylmethylamid hergestellt.
Ausbeute: 183 mg; LC/MS (Methode J): m/z = 498 (M+1); Rt = 0,758 min

### Beispiel 13

### Trennung des Diastereomerengemisches aus Beispiel 12

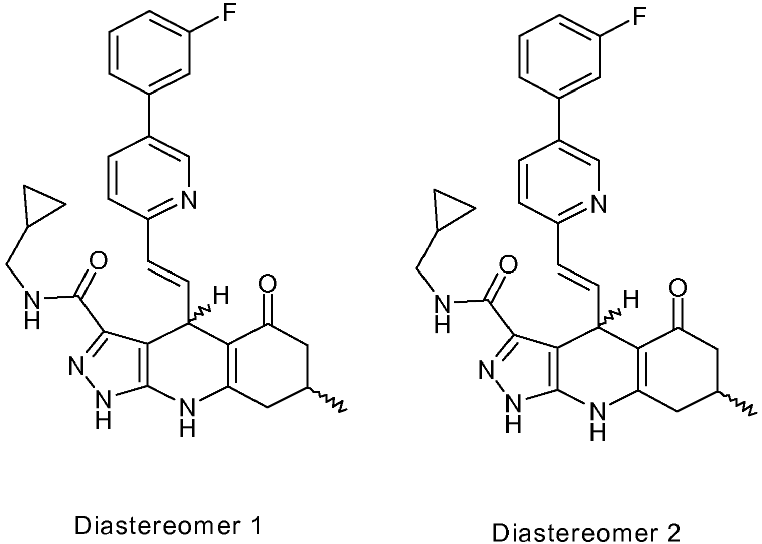

Das Diastereomerengemisch aus Beispiel 12 wird durch eine chirale Chromatografie (HPLC Säule: Chiralcel OD-H/61, 250 x 4,6 mm, Eluent: Heptan:Ethanol:MeOH 10:1:1, Flußrate: 1 ml/min, 30 °C) getrennt und lieferte 2 reine Diastereomere als Hauptprodukte von noch nicht bestimmter absoluter Konfiguration. Ausbeuten:
(Diastereomer 1): 54 mg; m/z = 498 (M+1); Rt = 8,349 min
(Diastereomer 2): 58 mg; m/z = 498 (M+1); Rt = 9,400 min

### Beispiel 14

### 4-{(E)-2-[5-(3-Fluor-phenyl)-pyridin-2-yl]-vinyl}-7,7-dimethyl-5-oxo-4,5,6,7,8,9-hexahydro-1H-pyrazolo[3,4-b]chinolin-3-carbonsäureethylester

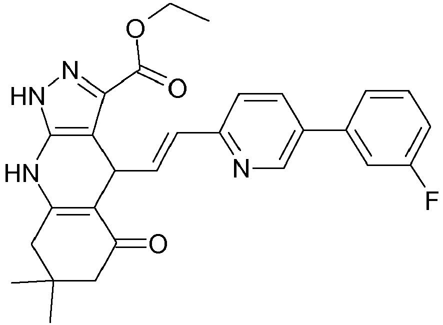

Die Titelverbindung wird als Diastereomerengemisch gemäß Arbeitsvorschrift A von 150 mg (E)-3-[5-(3-fluor-phenyl)-pyridin-2-yl]-propenal, 93 mg Dimedon und 102 mg 5-Amino-1 H-pyrazol-3-carbonsäureethylester hergestellt.
Ausbeute: 72 mg; LC/MS (Methode D): m/z = 487 (M+1); Rt = 1,115 min

### Beispiel 15

### 4-{(E)-2-[5-(3-Fluoro-phenyl)-pyridin-2-yl]-vinyl}-7,7-dimethyl-5-oxo-4,5,6,7,8,9-hexahydro-1H-pyrazolo[3,4-b]chinolin-3-carbonsäure-(2,2-dimethyl-propyl)amid

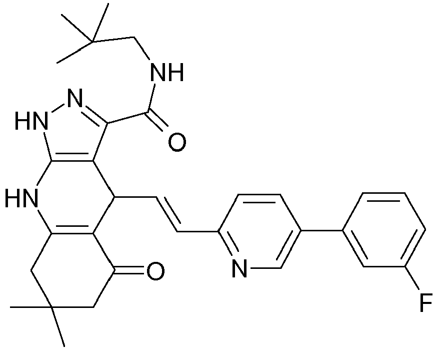

Die Titelverbindung wird gemäß Arbeitsvorschrift A von 174 mg (E)-3-[5-(3-fluor-phenyl)-pyridin-2-yl]-propenal, 107 mg Dimedon und 150 mg 5-Amino-1 H-pyrazol-3-carbonsäure-(2,2-dimethyl-propyl)-amid hergestellt.
Ausbeute: 102 mg; LC/MS (Methode J): m/z = 528 (M+1); Rt = 0,811 min

### Beispiel 16

### 4'-{(E)-2-[5-(3-Fluor-phenyl)-pyridin-2-yl]-vinyl}-5'-oxo-4',5',6',7',8',9'-hexahydro-spiro{cyclopentan-1,7-1H-pyrazolo[3,4-b]chinolin}-3'-carbonsäure-cyclopropylmethylamid

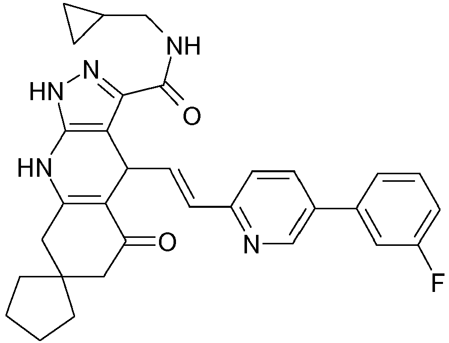

Die Titelverbindung wird gemäß Arbeitsvorschrift A von 151 mg (E)-3-[5-(3-fluor-phenyl)-pyridin-2-yl]-propenal, 111 mg Spiro[4.5]decan-7,9-dion und 120 mg 5-Amino-1H-pyrazol-3-carbonsäure-cyclopropylmethylamid hergestellt.
Ausbeute: 52 mg; LC/MS (Methode J): m/z = 538 (M+1); Rt = 0,808 min

### Beispiel 17

### 4-{(E)-2-[5-(3-Fluoro-phenyl)-pyridin-2-yl]-vinyl}-7,7-dimethyl-5-oxo-4,5,6,7,8,9-hexahydro-1H-pyrazolo[3,4-b]chinolin-3-carbonsäure-(1-ethyl-cyclobutylmethyl)-amid

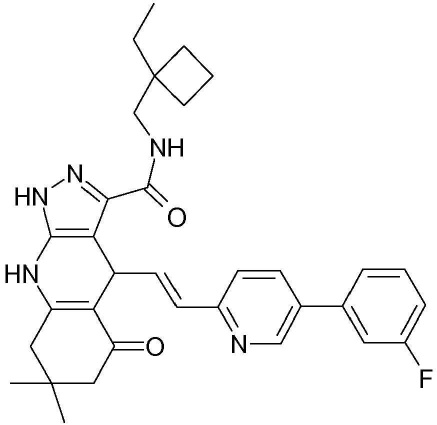

Die Titelverbindung wird gemäß Arbeitsvorschrift B von 123 mg (E)-3-[5-(3-fluor-phenyl)-pyridin-2-yl]-propenal, 76 mg Dimedon und 120 mg 5-Amino-1 H-pyrazol-3-carbonsäure-(1-ethyl-cyclobutylmethyl)amid hergestellt.
Ausbeute: 71 mg; LC/MS (Methode J): m/z = 554 (M+1); Rt = 0,855 min

### Beispiel 18

### 5-Amino-1H-pyrazol-3-carbonsäure-tert-butylester

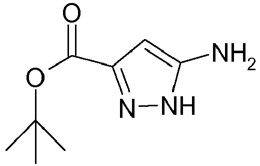

2 g 5-Nitro-3-pyrazolcarbonsäure wird in THF (5 mL) gelöst, dann mit Toluol (40 mL) verdünnt und am Rückfluss erhitzt. 10,35 g N,N-Dimethylformamid-di-tert-butylacetal wird zugetropft und die Mischung wird für 8 h unter Rückfluss erhitzt. Nach Abkühlen auf RT wird das Lösungsmittel unter verminderten Druck entfernt und der Rückstand in DCM aufgenommen. Es wird mit Wasser und zweimal mit gesät. NaHCO₃-Lösung gewaschen. Die organische Phase wird über Na₂SO₄ getrocknet und eingeengt. Der Rückstand wird durch Säulenchromatographie (Kieselgel, MeOH:DCM = 1-10:99-90) gereinigt. Der so erhaltene 1,26 g 5-Nitro-1H-pyrazol-3-carbonsäure-tert-butylester wird in MeOH (10 mL) gelöst und mit 88 mg Palladium auf Kohle (10%ig) versetzt. Es wird 2 h mit 4,5 bar Wasserstoffdruck hydriert. Die Lösung wird filtriert und das Lösungsmittel unter verminderten Druck entfernt. Ausbeute: 1,08 g; LC/MS (Methode J): m/z = 128 (M-tBu); Rt = 0,477 min

### Beispiel 19

### 4-{(E)-2-[5-(3-Fluoro-phenyl)-pyridin-2-yl]-vinyl}-7,7-dimethyl-5-oxo-4,5,6,7,8,9-hexahydro-1H-pyrazolo[3,4-b]chinolin-3-carbonsäure-tert-butylester

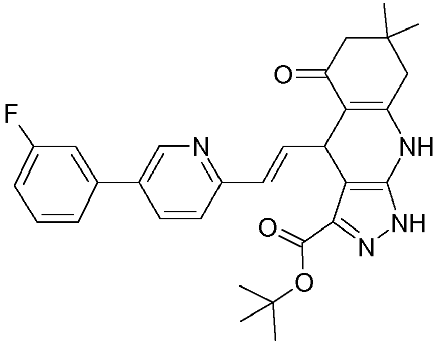

Die Titelverbindung wird gemäß Arbeitsvorschrift A von 620 mg (E)-3-[5-(3-fluor-phenyl)-pyridin-2-yl]-propenal, 383 mg Dimedon und 500 mg 5-Amino-1 H-pyrazol-3-carbonsäure- tert-butylester hergestellt.
Ausbeute: 149 mg; LC/MS (Methode J): m/z = 515 (M+1); Rt = 0,854 min

### Beispiel 20

### 4-{(E)-2-[5-(3-Fluoro-phenyl)-pyridin-2-yl]-vinyl}-7,7-dimethyl-5-oxo-4,5,6,7,8,9-hexahydro-1H-pyrazolo[3,4-b]chinolin-3-carbonsäure

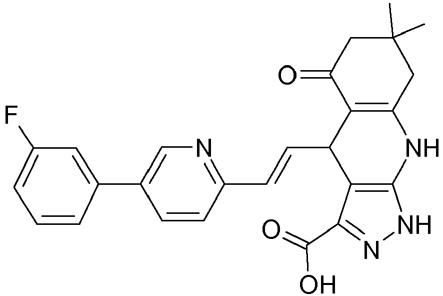

83 mg 4-{(E)-2-[5-(3-Fluoro-phenyl)-pyridin-2-yl]-vinyl}-7,7-dimethyl-5-oxo-4,5,6,7,8,9-hexahydro-1H-pyrazolo[3,4-b]chinolin-3-carbonsäure-tert-butylester werden in DCM (2,5 mL) gelöst, mit TFA (2,5 mL) versetzt und 3 h bei RT gerührt. Das Lösungsmittel wird unter verminderten Druck entfernt, der Rückstand in Toluol suspendiert und nochmals einrotiert. Der erhaltene Feststoff wird ohne Reinigung weiter eingesetzt.
Ausbeute: 120 mg; LC/MS (Methode J): m/z = 459 (M+1); Rt = 0,703 min

### Beispiel 21

### 4-{(E)-2-[5-(3-Fluoro-phenyl)-pyridin-2-yl]-vinyl}-7,7-dimethyl-5-oxo-4,5,6,7,8,9-hexahydro-1H-pyrazolo[3,4-b]chinolin-3-carbonsäure-(1-aminomethyl-cyclobutylmethyl)amid

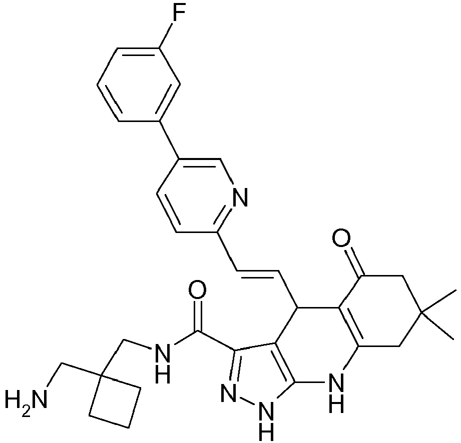

92 mg 4-{(E)-2-[5-(3-Fluoro-phenyl)-pyridin-2-yl]-vinyl}-7,7-dimethyl-5-oxo-4,5,6,7,8,9-hexahydro-1H-pyrazolo[3,4-b]chinolin-3-carbonsäure, 35 mg HOBt, 44 mg EDCI, 202 mg NMM und 75 mg C-(1-Aminomethyl-cyclobutyl)-methylamin-hydrochlorid werden in DMF (0,5 mL) suspendiert. Dann wird Wasser (160 µL) zugegeben und die nun entstandene Lösung 3 h bei RT gerührt. Die Reaktionsmischung wird mit ges. NaHCO₃-Lösung und Essigester versetzt. Die organische Phase wird abgetrennt und die wässrige Phase noch dreimal mit Essigester extrahiert. Die vereinigten organischen Phasen werden über Na₂SO₄ getrocknet und konzentriert. Der Rückstand wird chromatographisch gereinigt (Kieselgel, MeOH:DCM).
Ausbeute: 57 mg; LC/MS (Methode J): m/z = 555 (M+1); Rt = 0,682 min

### Beispiel 22

### {3-[(5-amine-1H-pyrazol-3-carbonyl)-amino]-2,2-dimethyl-propyl}-carbamidsäure-tert-butylester

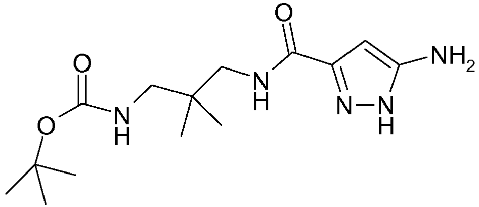

2,00 g 5-Nitro-3-pyrazolcarbonsäure, 2,24 g HOBt, 2,81 g EDCI, 3,86 g NMM und 2,58 g 1-Boc-Amino-2,2-Dimethyl-1,3-propandiamin werden in DMF (30 mL) gelöst und die Lösung 8 h bei RT gerührt. Die Reaktionsmischung wird mit ges. NaHCO₃-Lösung und Essigester versetzt. Die organische Phase wird abgetrennt und die wässrige Phase noch dreimal mit Essigester extrahiert. Die vereinigten organischen Phasen werden über Na₂SO₄ getrocknet und konzentriert. Der Rückstand wird chromatographisch gereinigt (Kieselgel, Essigester:Heptan). Der so erhaltenen 3,21 g {3-[(5-vitro-1H-pyrazol-3-carbonyl)-amino]-2,2-dimethyl-propyl}-carbamidsäure-tert-butylester wird in MeOH (20 mL) gelöst und mit 140 mg Palladium auf Kohle (10%ig) versetzt. Es wird 3 h mit 4,5 bar Wasserstoffdruck hydriert. Die Lösung wird filtriert und das Lösungsmittel unter verminderten Druck entfernt.
Ausbeute: 2,95 g; LC/MS (Methode J): m/z = 312 (M-tBu); Rt = 0,651 min

### Beispiel 23

### 4-{(E)-2-[5-(3-Fluoro-phenyl)-pyridin-2-yl]-vinyl}-7,7-dimethyl-5-oxo-4,5,6,7,8,9-hexahydro-1H-pyrazolo[3,4-b]chinolin-3-carbonsäure-(3-tert-butoxycarbonylamino-2,2-dimethylpropyl)ester

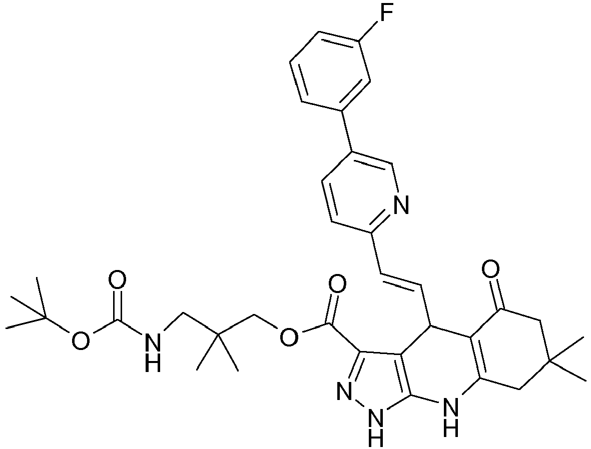

Die Titelverbindung wird gemäß Arbeitsvorschrift A von 110 mg (E)-3-[5-(3-fluor-phenyl)-pyridin-2-yl]-propenal, 68 mg Dimedon und 150 mg {3-[(5-Amino-1 H-pyrazol-3-carbonyl)-amino]-2,2-dimethyl-propyl}-carbamidsäure-tert-butylester hergestellt. Ausbeute: 113 mg; LC/MS (Methode J): m/z = 643 (M+1); Rt = 0,885 min

### Beispiel 24

### 4-{(E)-2-[5-(3-Fluoro-phenyl)-pyridin-2-yl]-vinyl}-7,7-dimethyl-5-oxo-4,5,6,7,8,9-hexahydro-1H-pyrazolo[3,4-b]chinolin-3-carbonsäure-(3-amino-2,2-dimethylpropyl)ester-Trifluoressigsäuresalz

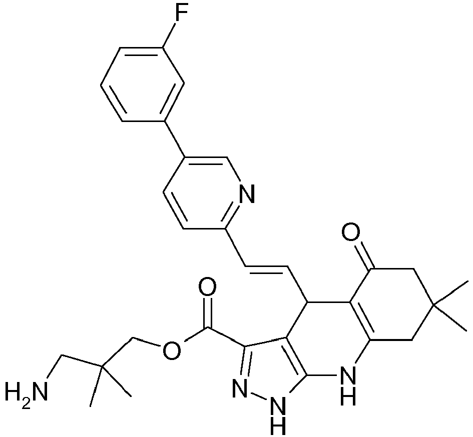

40 mg 4-{(E)-2-[5-(3-Fluoro-phenyl)-pyridin-2-yl]-vinyl}-7,7-dimethyl-5-oxo-4,5,6,7,8,9-hexahydro-1H-pyrazolo[3,4-b]chinolin-3-carbonsäure-(3-tert-butoxycarbonylamino-2,2-dimethylpropyl)ester werden in DCM (2,5 mL) gelöst, mit TFA (2,5 mL) versetzt und 3 h bei RT gerührt. Das Lösungsmittel wird unter verminderten Druck entfernt, der Rückstand in Toluol suspendiert und nochmals einrotiert. Ausbeute: 63 mg; LC/MS (Methode J): m/z = 543 (M+1); Rt = 0,680 min

### Beispiel 25

### 4-[(E)-2-(5-Brom-pyridin-2-yl)-vinyl]-7,7-dimethyl-5-oxo-4,5,6,7,8,9-hexahydro-1H-pyrazolo[3,4-b]chinolin-3-carbonsäure-cyclopropylmethylamid

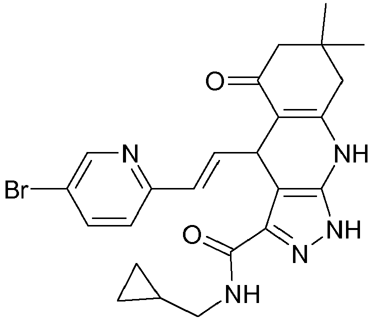

Die Titelverbindung wird analog Arbeitsvorschrift A von 150 mg (E)-3-(5-bromo-pyridin-2-yl)-propenal, 100 mg Dimedon und 127 mg 5-Amino-1 H-pyrazol-3-carbonsäure-cyclopropylmethylamid hergestellt.
Ausbeute: 133 mg; LC/MS (Methode J): m/z = 497 (M+1); Rt = 0,847 min

### Beispiel 26

### 4-[(E)-2-(5-Bromo-pyridin-2-yl)-vinyl]-7,7-dimethyl-5-oxo-4,5,6,7,8,9-hexahydro-1H-pyrazolo[3,4-b]chinolin-3-carbonsäure-isopropylamid

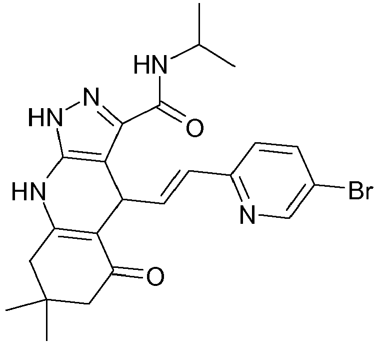

Die Titelverbindung wird analog Arbeitsvorschrift A von 240 mg (E)-3-(5-bromo-pyridin-2-yl)-propenal, 159 mg Dimedon und 190 mg 5-Amino-1 H-pyrazol-3-carbonsäure-isopropylamid hergestellt.
Ausbeute: 135 mg; LC/MS (Methode D): m/z = 485 (M+1); Rt = 1,168 min

### Beispiel 27

### 4-[(E)-2-(5-Brom-pyridin-2-yl)-vinyl]-5'-oxo-4',5',6',7',8',9'-hexahydro-spiro{cyclopentan-1,7-1H-pyrazolo[3,4-b]chinolin}-3'-carbonsäure-cyclopropylmethylamid

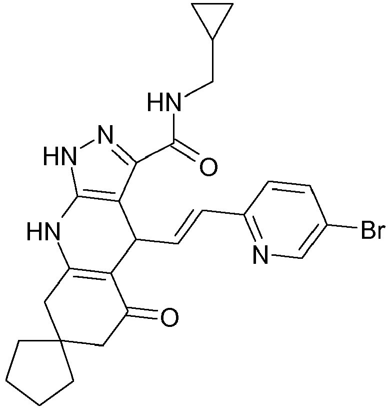

Die Titelverbindung wird analog Arbeitsvorschrift A von 470 mg (E)-3-(5-bromo-pyridin-2-yl)-propenal, 434 mg Spiro[4.5]decan-7,9-dion und 553 mg 5-Amino-1H-pyrazol-3-carbonsäure-cyclopropylmethylamid hergestellt.
Ausbeute: 825 mg; LC/MS (Methode J): m/z = 523 (M+1); Rt = 0,881 min

### Beispiel 28

### 4'-{(E)-2-[5-(3,5-Difluor-phenyl)-pyridin-2-yl]-vinyl}-5'-oxo-4',5',6',7',8',9'-hexahydro-spiro{cyclopentan-1,7-1H-pyrazolo[3,4-b]chinolin}-3'-carbonsäure-cyclopropylmethylamid

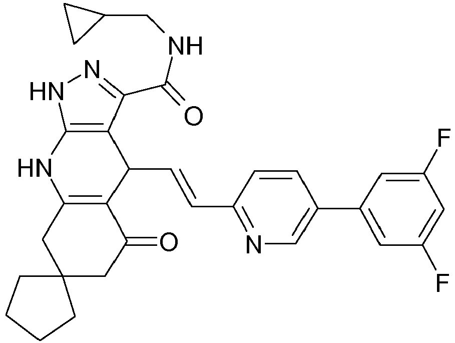

Die Titelverbindung wird gemäß Arbeitsvorschrift C von 150 mg 4-[(E)-2-(5-Brom-pyridin-2-yl)-vinyl]- 5'-oxo-4',5',6',7',8',9'-hexahydro-spiro{cyclopentan-1,7-1 H-pyrazolo[3,4-b]chinolin}-3'-carbonsäure-cyclopropylmethylamid, 68 mg 3,5-Difluorphenylboronsäure, 153 mg K₃PO₄ und 42 mg 1,1'-Bis(diphenylphosphino)ferrocen-palladiumdichlorid hergestellt.
Ausbeute: 47 mg; LC/MS (Methode J): m/z = 556 (M+1); Rt = 0,865 min

### Beispiel 29

### 4-{(E)-2-[5-(3,4-Difluoro-phenyl)-pyridin-2-yl]-vinyl}-7,7-dimethyl-5-oxo-4,5,6,7,8,9-hexahydro-1H-pyrazolo[3,4-b]chinolin-3-carbonsäure-cyclopropylmethylamid

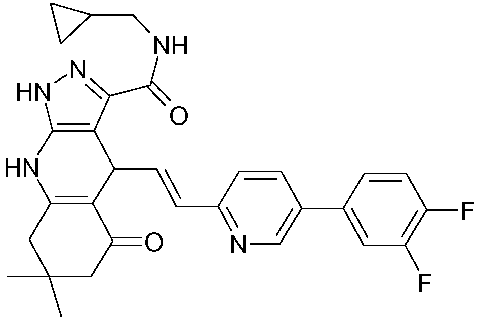

Die Titelverbindung wird gemäß Arbeitsvorschrift C von 125 mg 4-[(E)-2-(5-Brom-pyridin-2-yl)-vinyl]-7,7-dimethyl-5-oxo-4,5,6,7,8,9-hexahydro-1H-pyrazolo[3,4-b]chinolin-3-carbonsäure-cyclopropylmethylamid, 60 mg 3,4-Difluorphenyl-boronsäure, 134 mg K₃PO₄ und 37 mg 1,1'-Bis(diphenylphosphino)ferrocenpalladiumdichlorid hergestellt.
Ausbeute: 39 mg; LC/MS (Methode J): m/z = 530 (M+1); Rt = 0,566 min

Die Verbindungen in der nachfolgenden Tabelle 1 werden gemäß den Arbeitsvorschriften der vorstehenden Beispiele hergestellt.

**Tabelle 1**

| Beispiel | Strukturformel | Rt (aus LC/MS) [min] | Masse aus LC/MS (m/z) | LC/MS Methode | Herstell-Methode |
|---|---|---|---|---|---|
| 30 | 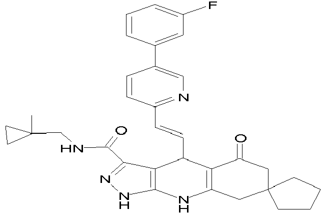 | 0,852 | 552 | J | B |
| 31 | 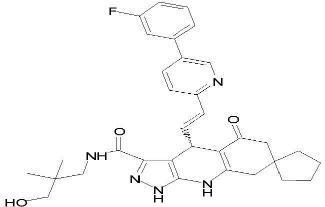 | 21,530 | 570 | B | B |
| 32 | 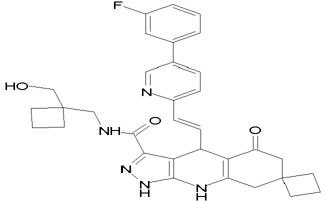 | 0,783 | 568 | J | B |
| 33 | 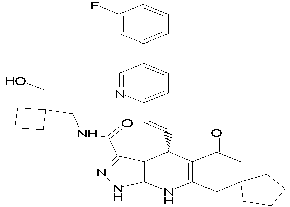 | 17,462 | 582 | C | B |
| 34 | 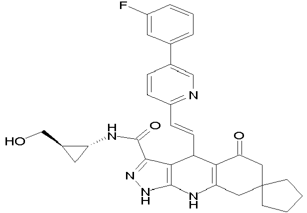 | 0,760 | 554 | J | B |
| 35 | 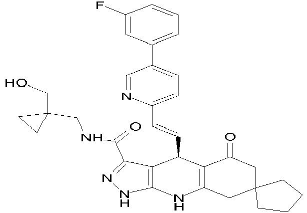 | 0,778 | 568 | J | B |
| 36 | 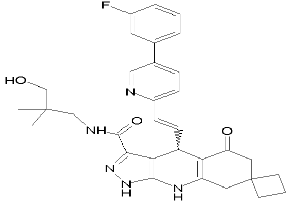 | 15,897 | 556 | C | B |
| 37 | 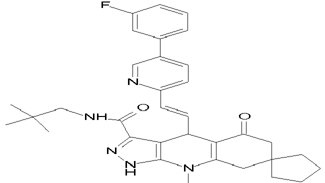 | 0,901 | 568 | J | B |
| 38 | 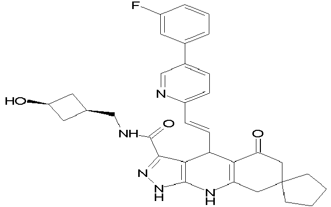 | 0,767 | 568 | J | B |
| 39 | 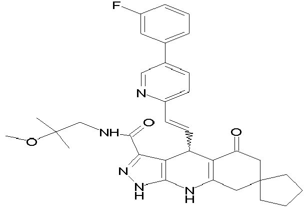 | 14,236 | 570 | E | B |
| 40 | 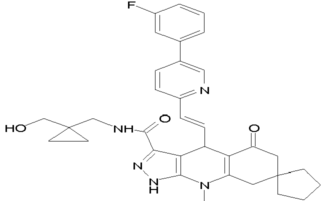 | 0,814 | 582 | J | B |
| 41 | 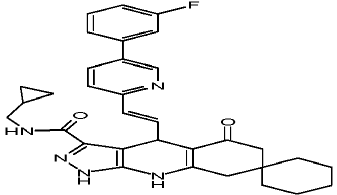 | 0,838 | 552 | J | A |
| 42 | 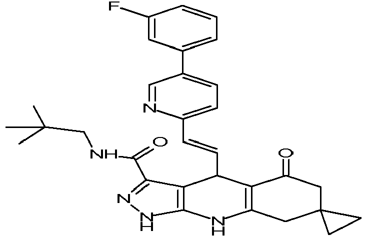 | 0,811 | 526 | J | A |
| 43 | 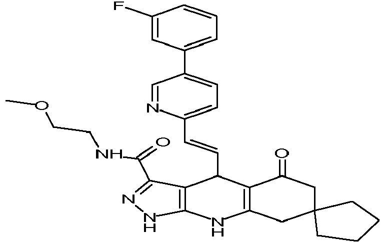 | 0,764 | 542 | J | B |
| 44 | 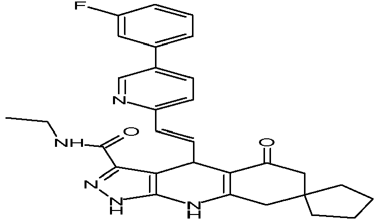 | 0,769 | 512 | J | B |
| 45 | 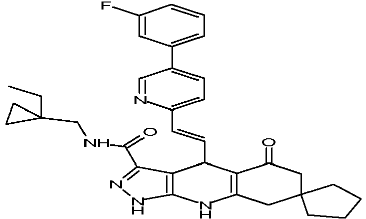 | 0,854 | 566 | J | B |
| 46 | 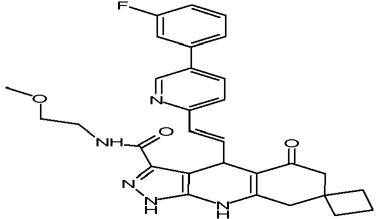 | 0,702 | 528 | J | B |
| 47 | 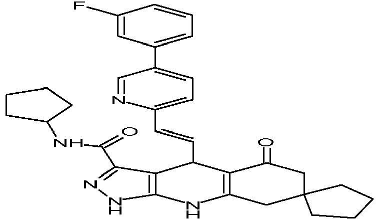 | 0,84 | 552 | J | B |
| 48 | 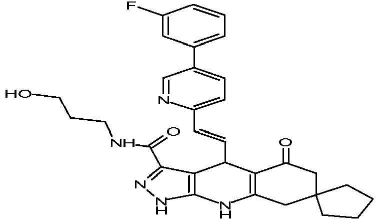 | 0,826 | 541 | J | B |
| 49 | 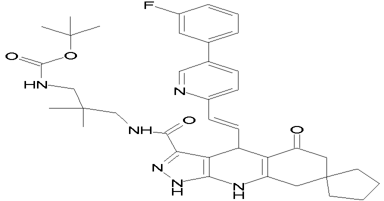 | 0,926 | 669 | J | B |
| 50 | 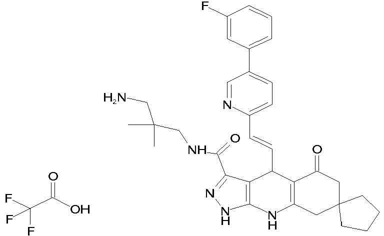 | 0,725 | 569 | J | B |
| 51 | 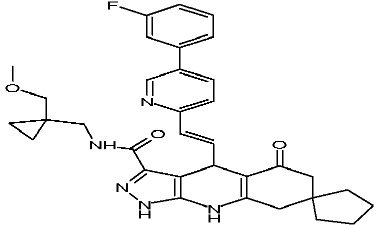 | 0,841 | 582 | J | B |
| 52 | 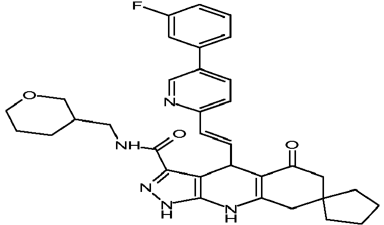 | 0,785 | 582 | J | B |
| 53 | 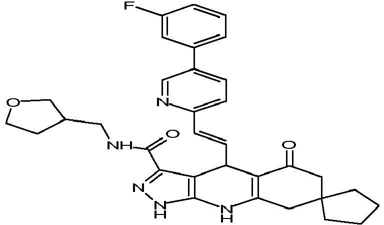 | 0,789 | 568 | J | B |
| 54 | 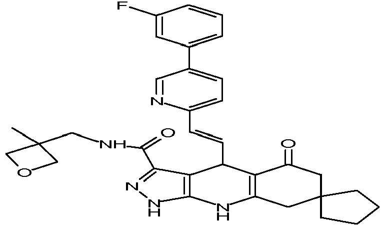 | 0,752 | 568 | J | B |
| 55 | 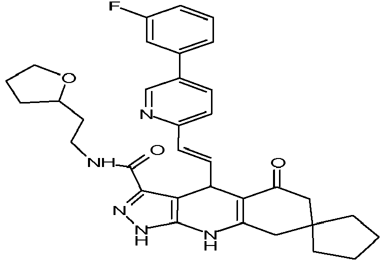 | 0,747 | 582 | J | B |

### Pharmakologische Beispiele

### PAR1-Bestimmungsmethode: Hemmung der PAR1-mediierten Thrombozytenaggregation

Die pharmakologische Testung der Substanzen erfolgte in der durch TRAP (Thrombinrezeptor-aktivierendes Peptid) induzierten Thrombozytenaggregation im 96-well Format. Dazu wurde von gesunden Freiwilligen Blut in 20 ml Spritzen abgenommen, in denen 2 ml 3,13 %-ige Natriumcitratlösung vorgelegt war. Nach einer 20-minütigen Zentrifugation bei 150 x g wurde das Plättchenreiche Plasma (PRP) abgetrennt und mit 1 µL PGE1-Lösung (500 µg/ml in Ethanol) / ml PRP versetzt. Nach 5 Minuten Inkubation bei RT wurde 15 Minuten bei 120 x g zentrifugiert um die Leukozyten zu entfernen. Das Leukozytenfreie PRP wurde in 5 ml Portionen in 15 ml PP Röhrchen überführt und 15 Minuten bei 360xg abzentrifugiert, um die Plättchen zu pelletieren. Anschließend wurde das Plasma dekantiert und das Plättchensediment aus 5 ml PRP in 1 ml Tyrode (120 mM NaCl, 2,6 mM KCl, 12 mM NaHCO₃, 0,39 mM NaH₂PO₄ x H₂O, 10 mM HEPES, 0,35% BSA (Bovine Serum Albumin), 5,5 mM Glukose, pH 7,4) resuspendiert und mit Tyrode auf eine Plättchenzahl von 3x10⁵ / Mikroliter (µL) eingestellt. 13 µL dieser Zellsuspension wurde dann mit 866 µL 10 mM CaCl₂-Lösung versetzt und 120 µL davon pro well einer 96-well-Platte pipettiert, in dem 15 µL der zu testenden Substanz vorgelegt waren. Nach 30 Minuten Inkubation bei RT im Dunklen wurden 15 µL einer TRAP-Lösung (70-100 µM) als Agonist zugegeben und in einem SpectraMax 340 bei 650 nm über 20 Minuten bei 37° C unter Schütteln eine Kinetik aufgezeichnet. Die Flächen unter den Kurven von Negativkontrolle (Tyrode/DMSO) und Positivkontrolle (15 µl Agonist/DMSO) wurden berechnet und die Differenz als 100 % Wert festgelegt. Die zu testenden Substanzen wurden in Doppelbestimmung als Verdünnungsreihen pipettiert, ebenfalls die AUC jeder Substanzkonzentration bestimmt und die % Hemmung der AUC gegen die Kontrolle errechnet. Anhand der % Hemmung wurde mit Hilfe nichtlinearer Regressionsanalyse gemäß der 4 Parameter-Gleichung die IC₅₀-Werte berechnet. Tabelle 2 zeigt Ergebnisse (IC₅₀-Werte in mikromol/L).

**Tabelle 2**

| Verbindung aus Beispiel | Hemmung der Thrombozytenaggregation IC₅₀ [mikro M] | Verbindung aus Beispiel | Hemmung der Thrombozytenaggregation IC₅₀ [mikro M] | Verbindung aus Beispiel | Hemmung der Thrombozytenaggregation IC₅₀ [mikro M] |
|---|---|---|---|---|---|
| 8 | 1,88 | 33 | <0,4 | 43 | 1,20 |
| 10 | 1,39 | 35 | 0,41 | 46 | 1,50 |
| 13 | 13,44 | 36 | 0,46 | 47 | 1,40 |
| 15 | 4,23 | 37 | 0,71 | 50 | 0,91 |
| Diastereomer 1 aus Beispiel 13 | 5,12 | 38 | 0,82 | 54 | 0,84 |
| 31 | 0,38 | 39 | 0,45 | | |

## Patentansprüche

1. Verbindung der Formel I, 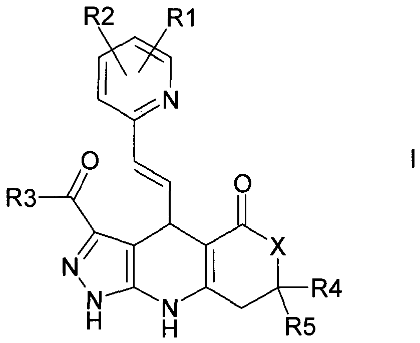 und/oder alle stereoisomeren oder tautomeren Formen der Verbindung der Formel I und/oder Gemische dieser Formen in jedem Verhältnis, und/oder ein physiologisch verträgliches Salz der Verbindung der Formel I, wobei
R1 für -(C₁-C₆)-Alkyl, Aryl, Halogen, oder für Hetaryl ausgewählt aus der Gruppe Thienyl, Pyrrolyl, Imidazolyl, Pyrazolyl, Pyridyl, Pyrimidinyl, Pyrazinyl oder Pyridazinyl stehen, wobei Alkyl, Aryl und Hetaryl jeweils unsubstituiert oder einfach, zweifach oder dreifach unabhängig voneinander durch F, Cl, Br, CN, -(C₁-C₄)-Alkyl, -O-(C₁-C₄)-Alkyl oder -CF₃ substituiert sind;
R2 für Wasserstoff steht,
R3 für-OH, -O-(C₁-C₈)-Alkyl, -O-(C₁-C₆)-Alkylen-NH-C(O)-O-(C₁-C₄)-Alkyl, -O-(C₁-C₆)-Alkylen-NH₂, -NH₂, -NH-(C₁-C₈)-Alkyl, -NH-(C₁-C₆)-Alkylen-Aryl, -NH-(C₁-C₈)-Alkylen-O-(C₁-C₄)-Alkyl, -NH-(C₁-C₈)-Alkyl-OH, -NH-(C₁-C₆)-Alkylen-NH-C(O)-O-(C₁-C₄)-Alkyl, -NH-(C₁-C₆)-Alkylen-NH₂, -NH-(C₀-C₆)-Alkylen-(C₃-C₇)-Cycloalkyl, -NH-(C₀-C₆)-Alkylen-Hetaryl, oder -N((C₁-C₄)-Alkyl)₂, wobei Aryl, Cycloalkyl und Hetaryl jeweils unsubstituiert oder einfach, zweifach oder dreifach unabhängig voneinander durch F, Cl, -OH, -(C₁-C₄)-Alkyl, -(C₁-C₄)-Alkylen-NH₂, -(C₁-C₄)-Alkylen-OH oder -O-(C₁-C₄)-Alkyl substituiert sind;
R4 und R5 gleich oder verschieden sind und unabhängig voneinander für Wasserstoff oder -(C₁-C₆)-Alkyl stehen, oder
R4 und R5 zusammen mit dem C-Atom, an das sie gebunden sind einen (C₃-C₇)-Cycloalkylrest bilden;
X für eine kovalente Bindung, CH₂, CH((C₁-C₆)-Alkyl), C((C₁-C₄)-Alkyl)₂ oder Sauerstoff steht;
wobei unter dem Begriff "Hetaryl" in R3 Ringsysteme verstanden werden mit 4 bis 15 Kohlenstoffatomen, die in ein, zwei oder drei miteinander verbundenen Ringsystemen vorliegen und die je nach Ringgröße ein, zwei, drei oder vier gleiche oder verschiedene Heteroatome aus der Reihe Sauerstoff, Stickstoff oder Schwefel enthalten oder wobei Hetaryl für einen Oxetanylrest steht.

2. Verbindung der Formel I gemäß Anspruch 1, und/oder alle stereoisomeren oder tautomeren Formen der Verbindung der Formel I und/oder Gemische dieser Formen in jedem Verhältnis, und/oder ein physiologisch verträgliches Salz der Verbindung der Formel I, wobei
R1 für -(C₁-C₆)-Alkyl, Aryl, Halogen oder für Hetaryl ausgewählt aus der Gruppe Thienyl, Pyrrolyl, Imidazolyl, Pyrazolyl, Pyridyl, Pyrimidinyl, Pyrazinyl oder Pyridazinyl stehen, wobei Alkyl, Aryl oder Hetaryl jeweils unsubstituiert oder einfach, zweifach oder dreifach unabhängig voneinander durch F, Cl, Br, CN, -(C₁-C₄)-Alkyl, -O-(C₁-C₄)-Alkyl oder -CF₃ substituiert sind;
R2 für Wasserstoff steht,
R3 für -O-(C₁-C₆)-Alkyl, -O-(C₁-C₆)-Alkylen-NH-C(O)-O-( C₁-C₄)-Alkyl, -O-(C₁-C₆)-Alkylen-NH₂, -NH-(C₁-C₆)-Alkyl, -NH-(C₁-C₆)-Alkylen-Aryl, -NH-(C₁-C₆)-Alkylen-(C₃-C₇)-Cycloalkyl, -NH-(C₁-C₆)-Alkylen-Hetaryl oder -N((C₁-C₄)-Alkyl)₂, wobei Aryl, Cycloalkyl und Hetaryl jeweils unsubstituiert oder einfach, zweifach oder dreifach unabhängig voneinander durch F, Cl, -OH, -(C₁-C₄)-Alkylen-NH₂, -(C₁-C₄)-Alkylen-OH oder -(C₁-C₄)-Alkyl substituiert sind;
R4 und R5 gleich oder verschieden sind und unabhängig voneinander für Wasserstoff oder -(C₁-C₆)-Alkyl stehen oder
R4 und R5 zusammen mit dem C-Atom, an das sie gebunden sind, (C₃-C₆)-Cycloalkyl bilden;
X für eine kovalente Bindung, CH₂, CH((C₁-C₄)-Alkyl), C((C₁-C₄)-Alkyl)₂ oder Sauerstoff steht;
wobei unter dem Begriff "Hetaryl" in R3 Ringsysteme verstanden werden mit 4 bis 15 Kohlenstoffatomen, die in ein, zwei oder drei miteinander verbundenen Ringsystemen vorliegen und die je nach Ringgröße ein, zwei, drei oder vier gleiche oder verschiedene Heteroatome aus der Reihe Sauerstoff, Stickstoff oder Schwefel enthalten oder wobei Hetaryl für einen Oxetanylrest steht.

3. Verbindung der Formel la gemäß Anspruch 1, 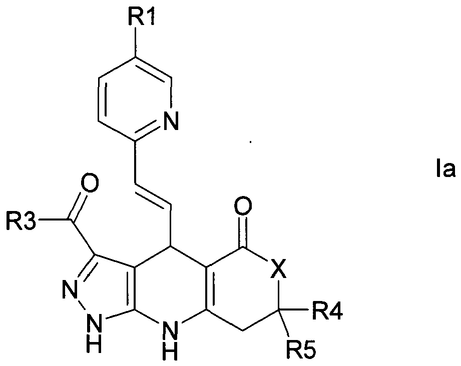 und/oder alle stereoisomeren oder tautomeren Formen der Verbindung der Formel la und/oder Gemische dieser Formen in jedem Verhältnis, und/oder ein physiologisch verträgliches Salz der Verbindung der Formel la, wobei
R1 für Wasserstoff, -(C₁-C₄)-Alkyl, Phenyl, Cl, Br oder für Hetaryl ausgewählt aus der Gruppe Thienyl, Pyrrolyl, Imidazolyl, Pyrazolyl, Pyridyl, Pyrimidinyl, Pyrazinyl oder Pyridazinylsteht, und wobei Phenyl und Hetaryl jeweils unsubstituiert oder einfach oder zweifach unabhängig voneinander durch F, Br, Cl, CN, -(C₁-C₄)-Alkyl, - O-(C₁-C₄)-Alkyl oder -CF₃ substituiert sind;
R3 fü r -OH, -O-(C₁-C₆)-Alkyl, -O-(C₁-C₆)-Alkylen-NH-C(O)-O-(C₁-C₄)-Alkyl, -O-(C₁-C₆)-Alkylen-NH₂, -NH-(C₁-C₄)-Alkyl, -NH-Benzyl oder -NH-Methylen-(C₃-C₆)-Cycloalkyl steht, wobei Cycloalkyl unsubstituiert oder einfach durch -(C₁-C₄)-Alkyl, -(C₁-C₄)-Alkylen-NH₂ oder -CH₂-OH substituiert ist;
R4 und R5 gleich oder verschieden sind und unabhängig voneinander für Wasserstoff oder -(C₁-C₄)-Alkyl stehen, oder
R4 und R5 zusammen mit dem C-Atom, an das sie gebunden sind, Cyclopropyl, Cyclobutyl, Cyclopentyl oder Cyclohexyl bilden;
X für eine kovalente Bindung, CH₂, C((C₁-C₄)-Alkyl)₂ oder Sauerstoff steht.

4. Verbindung der Formel la gemäß Anspruch 3, und/oder alle stereoisomeren oder tautomeren Formen der Verbindung der Formel la und/oder Gemische dieser Formen in jedem Verhältnis, und/oder ein physiologisch verträgliches Salz der Verbindung der Formel la, wobei
R1 für -(C₁-C₄)-Alkyl, Phenyl, Cl oder Br steht, wobei Phenyl unsubstituiert oder einfach, zweifach oder dreifach unabhängig voneinander durch F, Cl, Br, CN, -(C₁-C₄)-Alkyl, -O-(C₁-C₄)-Alkyl oder -CF₃ substituiert ist;
R3 für -OH, -O-(C₁-C₄)-Alkyl, -O-(C₁-C₆)-Alkylen-NH-C(O)-O-(C₁-C₄)-Alkyl, -O-(C₁-C₆)-Alkylen-NH₂, -NH-(C₁-C₃)-Alkyl oder -NH-Methylen-(C₃-C₆)-Cycloalkyl steht, wobei Cycloalkyl unsubstituiert oder einfach durch -(C₁-C₄)-Alkyl, -(C₁-C₄)-Alkylen-NH₂ oder -CH₂-OH substituiert ist;
R4 und R5 gleich oder verschieden sind und unabhängig voneinander für Wasserstoff oder -(C₁-C₄)-Alkyl stehen, oder
R4 und R5 zusammen mit dem C-Atom, an das sie gebunden sind, Cyclobutyl oder Cyclopentyl bilden;
X für eine kovalente Bindung, CH₂, C((C₁-C₄)-Alkyl)₂ oder Sauerstoff steht.

5. Verbindung der Formel I oder la gemäß den Ansprüchen 1 bis 4, und/oder alle stereoisomeren oder tautomeren Formen der Verbindung der Formel I oder la und/oder Gemische dieser Formen in jedem Verhältnis, und/oder ein physiologisch verträgliches Salz der Verbindung der Formel I oder la, wobei die Verbindung der Formel I oder la ausgewählt ist aus der Gruppe
4-{(E)-2-[5-(3-Fluor-phenyl)-pyridin-2-yl]-vinyl}-7,7-dimethyl-5-oxo-4,5,6,7,8,9-hexahydro-1H-pyrazolo[3,4-b]chinolin-3-carbonsäure-cyclopropylmethylamid,
7,7-Dimethyl-4-[(E)-2-(6-methyl-pyridin-2-yl)-vinyl]-5-oxo-4,5,6,7,8,9-hexahydro-1H-pyrazolo[3,4-b]chinolin-3-carbonsäure-isopropylamid,
4-{(E)-2-[5-(3-Fluor-phenyl)-pyridin-2-yl]-vinyl}-7,7-dimethyl-5-oxo-4,5,6,7,8,9-hexahydro-1H-pyrazolo[3,4-b]chinolin-3-carbonsäure-isopropylamid,
4-{(E)-2-[5-(3-Fluor-phenyl)-pyridin-2-yl]-vinyl}-6,6-dimethyl-5-oxo-4,5,6,7,8,9-hexahydro-1H-pyrazolo[3,4-b]chinolin-3-carbonsäure-cyclopropylmethylamid,
4-{(E)-2-[5-(3-Fluor-phenyl)-pyridin-2-yl]-vinyl}-7-isopropyl-5-oxo-4,5,6,7,8,9-hexahydro-1H-pyrazolo[3,4-b]chinolin-3-carbonsäure-cyclopropylmethylamid,
4-[(E)-2-(5-Brom-pyridin-2-yl)-vinyl]-7,7-dimethyl-5-oxo-4,5,6,7,8,9-hexahydro-1H-pyrazolo[3,4-b]chinolin-3-carbonsäure-cyclopropylmethylamid,
4-[(E)-2-(5-Bromo-pyridin-2-yl)-vinyl]-7,7-dimethyl-5-oxo-4,5,6,7,8,9-hexahydro-1H-pyrazolo[3,4-b]chinolin-3-carbonsäure-isopropylamid,
4-{(E)-2-[5-(3-Fluor-phenyl)-pyridin-2-yl]-vinyl}-7-methyl-5-oxo-4,5,6,7,8,9-hexahydro-1H-pyrazolo[3,4-b]chinolin-3-carbonsäure-isopropylamid,
4-{(E)-2-[5-(3-Fluor-phenyl)-pyridin-2-yl]-vinyl}-7-methyl-5-oxo-4,5,6,7,8,9-hexahydro-1H-pyrazolo[3,4-b]chinolin-3-carbonsäure-cyclopropylmethylamid,
4-{(E)-2-[5-(3-Fluor-phenyl)-pyridin-2-yl]-vinyl}-7,7-dimethyl-5-oxo-4,5,6,7,8,9-hexahydro-1H-pyrazolo[3,4-b]chinolin-3-carbonsäure-ethylester, und
4-{(E)-2-[5-(3-Fluoro-phenyl)-pyridin-2-yl]-vinyl}-7,7-dimethyl-5-oxo-4,5,6,7,8,9-hexahydro-1H-pyrazolo[3,4-b]chinolin-3-carbonsäure-(2,2-dimethyl-propyl)amid.

6. Arzneimittel, **gekennzeichnet durch** einen wirksamen Gehalt an mindestens einer Verbindung der Formel I oder la oder eines physiologisch verträglichen Salzes davon gemäß einem oder mehreren der Ansprüche 1 bis 5 zusammen mit einem pharmazeutisch geeigneten und physiologisch verträglichen Trägerstoff, Zusatzstoff und/oder anderen Wirkstoffen und Hilfsstoffen.

7. Verbindung der Formel I oder la oder ein physiologisch verträgliches Salz davon gemäß einem oder mehreren der Ansprüche 1 bis 5 zur Verwendung als Pharmazeutikum.

8. Verbindung der Formel I oder la oder ein physiologisch verträgliches Salz davon gemäß einem oder mehreren der Ansprüche 1 bis 5 zur Verwendung für die Prophylaxe, Sekundärprevention und Therapie all solcher Erkrankungen, die mit Thrombosen, Embolien, Hyperkoagulabilität, fibrotischen Veränderungen oder entzündlichen Erkrankungen einhergehen.

9. Verbindung der Formel I oder la oder ein physiologisch verträgliches Salz davon gemäß einem oder mehreren der Ansprüche 1 bis 5 zur Verwendung gemäß Anspruch 8, **dadurch gekennzeichnet, dass** es sich um Myokardinfarkt, Angina pectoris und andere Formen des akuten Koronarsyndroms, den akuten Schlaganfall oder dessen Sekundärprevention, die peripher vaskulären Erkrankungen, die tiefe Venenthrombose, die Lungenembolie, embolische oder thrombotische Ereignisse bedingt durch kardiale Arrhythmien, kardiovaskuläre Ereignisse wie Restenose nach Revaskularisierung und Angioplastie und ähnlichen Eingriffen wie Stentimplantationen und Bypass-Operationen, die Reduktion der Thrombosegefahr nach chirurgischen Eingriffen wie bei Knieoperationen und Hüftgelenksoperationen, Eingriffe, die zu einem Kontakt des Blutes mit Fremdoberflächen führen wie bei Dialysepatienten und Patienten mit Verweilkathetern, die disseminierte intravaskuläre Koagulation, Sepsis und andere intravaskuläre Ereignisse, die mit einer Entzündung einhergehen, Atherosklerose, Diabetes und das metabolische Syndrom und deren Folgen, Tumorwachstum und Tumormetastasierung, entzündliche und degenerative Gelenkserkrankungen wie die rheumatoide Arthritis und die Arthrose, Störungen des hämostatischen Systems wie Fibrinablagerungen, fibrotische Veränderungen der Lunge wie die chronische obstruktive Lungenerkrankung, das adult respiratory distress syndrom, Fibrinablagerungen des Auges nach Augenoperationen, oder die Verhinderung und/oder Behandlung von Narbenbildung handelt.

10. Verfahren zur Herstellung einer Verbindung der Formel I oder Ia oder eines physiologisch verträglichen Salzes davon gemäß einem oder mehreren der Ansprüche 1 bis 5, **dadurch gekennzeichnet, dass** man
a) eine Verbindung der Formel II, 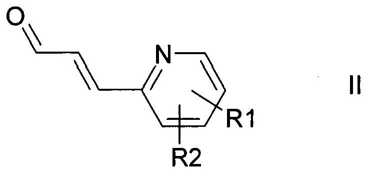 wobei die Reste R1, R2 wie in Formel I oder la definiert sind, mit einer Verbindung der Formel III und einer Verbindung der Formel IV, 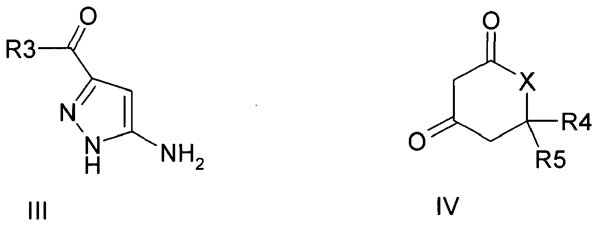 wobei die Reste R1, R2 wie in Formel I oder la definiert sind, in Gegenwart eines geeigneten Lösungsmittels oder Lösungsmittelgemisches bei 20°C bis 120°C zu einer Verbindung der Formel I oder la umsetzt; oder
b) eine Verbindung der Formel V, 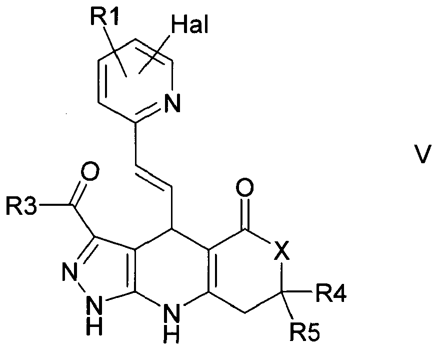 wobei die Reste X, R1, R3, R4 und R5 wie in Formel I oder la definiert sind und Hal die Bedeutung Chlor, Brom, Iod oder Triflat hat, mit einer Verbindung der Formel R2-B(OH)₂ in Gegenwart einer Base und eines geeigneten Metallkatalysators in einem geeigneten Lösemittel oder Lösemittelgemisch zu einer Verbindung der Formel I oder II umsetzt; oder
c) eine Verbindung der Formel I, worin X die Bedeutung NH hat, mit einem geeigneten Alkylierungsmittel in Gegenwart einer Base und in einem geeigneten inerten Lösemittel, bei Raumtemperatur oder bei erhöhter Temperatur zu einer Verbindung der Formel I umsetzt, worin X die Bedeutung N-R6 hat und R6 -(C₁-C₆)-Alkyl, -(C₁-C₆)-Alkylen-(C₃-C₇)-Cycloalkyl oder -(C₁-C₆)-Alkylen-Aryl bedeutet; oder
d) eine Verbindung der Formel I oder Ia, worin R3 für -OH, -O-Aryl oder -O-(C₁-C₆)-Alkyl steht und R1, R2, R4, R5 und X wie in Formel I oder la definiert sind, nach gängigen Verfahren zu einer Verbindung der Formel I oder la umsetzt, worin R3 für -NH₂, -NH-(C₁-C₆)-Alkyl, -NH-(C₁-C₆)-Alkylen-Aryl, -NH-(C₁-C₆)-Alkylen-(C₃-C₇)-Cycloalkyl, -NH-(C₁-C₆)-Alkylen-Hetaryl, -N((C₁-C₄)-Alkyl)₂ oder ein cyclisches Amin steht, das aus der Gruppe Hexamethylenimin, Morpholin, Piperazin, Piperidin, Pyrrolidin und Thiomorpholin ausgewählt ist und über das Stickstoffatom gebunden ist, wobei Aryl, Hetaryl und cyclisches Amin jeweils unsubstituiert oder einfach, zweifach oder dreifach unabhängig voneinander durch F, Cl, -OH, -(C₁-C₄)-Alkyl, -O-(C₁-C₄)-Alkyl, Aryl oder Hetaryl substituiert sind; oder
e) die nach den Verfahren a) bis d) hergestellte Verbindung der Formel I oder la, oder eine geeignete Vorstufe der Verbindung der Formel I oder Ia, die aufgrund ihrer chemischen Struktur in enantiomeren oder diastereomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren oder Diastereomeren auftrennt; oder
f) die nach den Verfahren a) bis e) hergestellte Verbindung der Formel I oder la entweder in freier Form isoliert, oder aus einem physiologisch unverträglichen Salz freisetzt, oder im Falle des Vorliegens von sauren oder basischen Gruppen in ein physiologisch verträgliches Salz umwandelt.

## Claims

1. A compound of the formula I 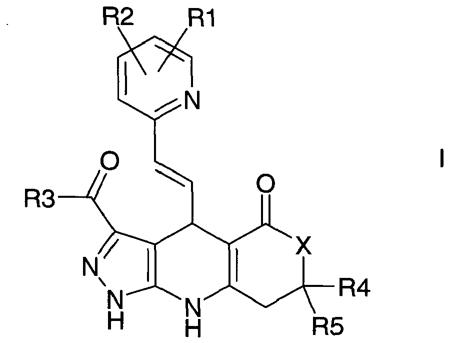 and/or all stereoisomeric or tautomeric forms of the compound of the formula I and/or mixtures of these forms in any ratio, and/or a physiologically acceptable salt of the compound of the formula I, where
R1 is -(C₁-C₆)-alkyl, aryl, halogen or is hetaryl selected from the group consisting of thionyl, pyrrolyl, imidazolyl, pyrazolyl, pyridyl, pyrimidinyl, pyrazinyl or pyridazinyl, where alkyl, aryl and hetaryl are in each case unsubstituted or mono-, di- or trisubstituted independently of one another by F, Cl, Br, CN, - (C₁-C₄) -alkyl, -O-(C₁-C₄)-alkyl or -CF₃ ;
R2 is hydrogen,
R3 is OH, -O-(C₁-C₈)-alkyl, -O-(C₁-C₆)-alkylene-NH-C(O) - O-(C₁-C₄)-alkyl, -O-(C₁-C₆)-alkylene-NH₂, -NH₂, -NH-(C₁-C₈)-alkyl, -NH-(C₁-C₆)-alkylenearyl, -NH-(C₁-C₈)-alkylene-O-(C₁-C₄)-alkyl, -NH-(C₁-C₈)-alkyl-OH, -NH-(C₁-C₆)-alkylene-NH-C(O)-O- (C₁-C₄)-alkyl, -NH-(C₁-C₆)-alkylene-NH₂, -NH- (C₀-C₆) -alkylene- (C₃-C₇) -cycloalkyl, -NH-(C₀-C₆)-alkylenehetaryl, or -N((C₁-C₄)-alkyl)₂, where aryl, cycloalkyl and hetaryl are in each case unsubstituted or mono-, di- or trisubstituted independently of one another by F, Cl, -OH, -(C₁-C₄)-alkyl, -(C₁-C₄) -alkylene-NH₂, -(C₁-C₄) -alkylene-OH or -O-(C₁-C₄) -alkyl;
R4 and R5 are identical or different and are independently of one another hydrogen or -(C₁-C₆)-alkyl,
or
R4 and R5 together with the carbon atom to which they are attached form a (C₃-C₇)-cycloalkyl radical;
X is a covalent bond, CH₂, CH((C₁-C₆)-alkyl), C((C₁-C₄)-alkyl)₂ or oxygen;
where the term "hetaryl" in R3 means ring systems having 4 to 15 carbon atoms which are present in one, two or three ring systems connected together, and which comprise, depending on the ring size, one, two, three or four identical or different heteroatoms from the group consisting of oxygen, nitrogen and sulfur or where hetaryl is an oxetanyl radical.

2. The compound of the formula I as claimed in claim 1, and/or all stereoisomeric or tautomeric forms of the compound of the formula I and/or mixtures of these forms in any ratio, and/or a physiologically acceptable salt of the compound of the formula I, where R1 is -(C₁-C₆)-alkyl, aryl, halogen or is hetaryl selected from the group consisting of thionyl, pyrrolyl, imidazolyl, pyrazolyl, pyridyl, pyrimidinyl, pyrazinyl or pyridazinyl, where alkyl, aryl or hetaryl are in each case unsubstituted or mono-, di- or trisubstituted independently of one another by F, Cl, Br, CN, -(C₁-C₄)-alkyl, -O-(C₁-C₄)-alkyl or -CF₃ ;
R2 is hydrogen,
R3 is -O-(C₁-C₆)-alkyl, -O-(C₁-C₆)-alkylene-NH-C(O)-O-(C₁-C₄)-alkyl, -O-(C₁-C₆)-alkyl ene-NH₂, -NH-(C₁-C₆)-alkyl, -NH- (C₁-C₆) -alkylenearyl -NH- (C₁-C₆) -alkylene- (C₃-C₇)-cycloalkyl, -NH-(C₁-C₆)-alkylenehetaryl or -N((C₁-C₄)-alkyl)₂, where aryl, cycloalkyl and hetaryl are in each case unsubstituted or mono-, di- or trisubstituted independently of one another by F, Cl, -OH, -(C₁-C₄)-alkylene-NH₂, -(C₁-C₄)-alkylene-OH or -(C₁-C₄)-alkyl;
R4 and R5 are identical or different and are independently of one another hydrogen or -(C₁-C₆)-alkyl,
or
R4 and R5 together with the carbon atom to which they are attached form (C₃-C₆)-cycloalkyl;
X is a covalent bond, CH₂, CH((C₁-C₄)-alkyl), C((C₁-C₄)-alkyl)₂ or oxygen;
where the term "hetaryl" in R3 means ring systems having 4 to 15 carbon atoms which are present in one, two or three ring systems connected together, and which may, depending on the ring size, comprise one, two, three or four identical or different heteroatoms from the group consisting of oxygen, nitrogen and sulfur or where hetaryl is an oxetanyl radical.

3. The compound of the formula Ia as claimed in claim 1, 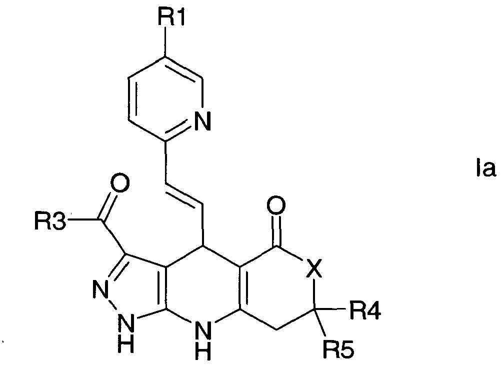 and/or all stereoisomeric or tautomeric forms of the compound of the formula Ia and/or mixtures of these forms in any ratio, and/or a physiologically acceptable salt of the compound of the formula Ia, where
R1 is hydrogen, -(C₁-C₄)-alkyl, phenyl, Cl, Br or is hetaryl selected from the group consisting of thienyl, pyrrolyl, imidazolyl, pyrazolyl, pyridyl, pyrimidinyl, pyrazinyl or pyridazinyl, and where phenyl and hetaryl are in each case unsubstituted or mono- or disubstituted independently of one another by F, Br, Cl, CN, -(C₁-C₄)-alkyl , -O-(C₁-C₄)-alkyl or -CF₃ ;
R3 is OH, -O-(C₁-C₆)-alkyl, -O-(C₁-C₆)-alkylene-NH-C(O)-O-(C₁-C₄)-alkyl, -O-(C₁-C₆)-alkylene-NH₂, -NH-(C₁-C₄)-alkyl, -NH-benzyl or -NH-methylene-(C₃-C₆)-cycloalkyl, where cycloalkyl is unsubstituted or monosubstituted by -(C₁-C₄)-alkyl, -(C₁-C₄)-alkylene-NH₂ or -CH₂-OH;
R4 and R5 are identical or different and are independently of one another hydrogen or -(C₁-C₄)-alkyl,
or
R4 and R5 together with the carbon atom to which they are attached form cyclopropyl, cyclobutyl, cyclopentyl or cyclohexyl;
X is a covalent bond, CH₂, C((C₁-C₄)-alkyl)₂ or oxygen.

4. The compound of the formula Ia as claimed in claim 3, and/or all stereoisomeric or tautomeric forms of the compound of the formula Ia and/or mixtures of these forms in any ratio, and/or a physiologically acceptable salt of the compound of the formula Ia, where
R1 is -(C₁-C₄)-alkyl, phenyl, Cl or Br, where phenyl is unsubstituted or mono-, di- or trisubstituted independently of one another by F, Cl, Br, CN, -(C₁-C₄)-alkyl, -O-(C₁-C₄)-alkyl or -CF₃ ;
R3 is OH, -O-(C₁-C₄)-alkyl, -O-(C₁-C₆)-alkylene-NH-C(O)-O-(C₁-C₄)-alkyl , -O-(C₁-C₆)-alkylene-NH₂, -NH-(C₁-C₃)-alkyl or -NH-methylene-(C₃-C₆)- cycloalkyl, where cycloalkyl is unsubstituted or monosubstituted by -(C₁-C₄)-alkyl, -(C₁-C₄)-alkylene-NH₂ or -CH₂-OH;
R4 and R5 are identical or different and are independently of one another hydrogen or -(C₁-C₄)-alkyl,
or
R4 and R5 together with the carbon atom to which they are attached form cyclobutyl or cyclopentyl;
X is a covalent bond, CH₂, C((C₁-C₄)-alkyl)₂ or oxygen.

5. The compound of the formula I or Ia as claimed in claims 1 to 4, and/or all stereoisomeric or tautomeric forms of the compound of the formula I or Ia and/or mixtures of these forms in any ratio, and/or a physiologically acceptable salt of the compound of the formula I or Ia, where the compound of the formula I or Ia is selected from the group consisting of
4-{(E)-2-[5-(3-fluorophenyl)pyridin-2-yl]vinyl}-7,7-dimethyl-5-oxo-4,5,6,7,8,9-hexahydro-1H-pyrazolo[3,4-b]quinoline-3-cyclopropylmethylcarboxamide,
7,7-dimethyl-4-[(E)-2-(6-methylpyridin-2-yl)vinyl]-5-oxo-4,5,6,7,8,9-hexahydro-1H-pyrazolo[3,4-b]quinoline-3-isopropylcarboxamide,
4-{(E)-2-[5-(3-fluorophenyl)pyridin-2-yl]vinyl}-7,7-dimethyl-5-oxo-4,5,6,7,8,9-hexahydro-1H-pyrazolo-[3,4-b]quinoline-3-isopropylcarboxamide,
4-{(E)-2-[5-(3-fluorophenyl)pyridin-2-yl]vinyl}-6,6-dimethyl-5-oxo-4,5,6,7,8,9-hexahydro-1H-pyrazolo-[3,4-b]quinoline-3-cyclopropylmethylcarboxamide,
4-{(E)-2-[5-(3-fluorophenyl)pyridin-2-yl]vinyl}-7-isopropyl-5-oxo-4,5,6,7,8,9-hexahydro-1H-pyrazolo-[3,4-b]quinoline-3-cyclopropylmethylcarboxamide,
4-[(E)-2-(5-bromopyridin-2-yl)vinyl]-7,7-dimethyl-5-oxo-4,5,6,7,8,9-hexahydro-1H-pyrazolo[3,4-b]quinoline-3-cyclopropylmethylcarboxamide,
4-[(E)-2-(5-bromopyridin-2-yl)vinyl]-7,7-dimethyl-5-oxo-4,5,6,7,8,9-hexahydro-1H-pyrazolo[3,4-b]quinoline-3-isopropylcarboxamide,
4-{(E)-2-[5-(3-fluorophenyl)pyridin-2-yl]vinyl}-7-methyl-5-oxo-4,5,6,7,8,9-hexahydro-1H-pyrazolo-[3,4-b]quinoline-3-isopropylcarboxamide,
4-{(E)-2-[5-(3-fluorophenyl)pyridin-2-yl]vinyl}-7-methyl-5-oxo-4,5,6,7,8,9-hexahydro-1H-pyrazolo-[3,4-b]quinoline-3-cyclopropylmethylcarboxamide, ethyl 4-{(E)-2-[5-(3-fluorophenyl)pyridin-2-yl]vinyl}-7,7-dimethyl-5-oxo-4,5,6,7,8,9-hexahydro-1H-pyrazolo-[3,4-b]quinoline-3-carboxylate, and
4-{(E)-2-[5-(3-fluorophenyl)pyridin-2-yl]vinyl}-7,7-dimethyl-5-oxo-4,5,6,7,8,9-hexahydro-1H-pyrazolo-[3,4-b]quinoline-3-(2,2-dimethylpropyl)carboxamide.

6. A medicament having an effective content of at least one compound of the formula I or Ia or a physiologically acceptable salt thereof as claimed in one or more of claims 1 to 5 together with a pharmaceutically suitable and physiologically acceptable carrier, additive and/or other active compounds and auxiliaries.

7. The compound of the formula I or Ia or a physiologically acceptable salt thereof as claimed in one or more of claims 1 to 5 for use as pharmaceutic.

8. The compound of the formula I or Ia or a physiologically acceptable salt thereof as claimed in one or more of claims 1 to 5 for use for the prophylaxis, secondary prevention and therapy of all disorders associated with thromboses, embolisms, hypercoagulability, fibrotic changes or inflammatory disorders.

9. The compound of the formula I or Ia or a physiologically acceptable salt thereof as claimed in one or more of claims 1 to 5 for the use as claimed in claim 8, wherein the use is for myocardial infarction, angina pectoris and other types of acute coronary syndrome, acute stroke or its secondary prevention, peripheral vascular disorders, deep vein thrombosis, pulmonary embolism, embolic or thrombotic events caused by cardiac arrhythmias, cardiovascular events such as restenosis following revascularization and angioplasty and similar procedures such as stent implantations and bypass operations, reduction of the risk of thrombosis following surgical procedures such as knee and hip joint operations, procedures leading to contact of blood with foreign surfaces, such as for dialysis patients and patients with indwelling catheters, disseminated intravascular coagulation, sepsis and other intravascular events associated with inflammation, atherosclerosis, diabetes and the metabolic syndrome and the sequelae thereof, tumor growth and tumor metastasis, inflammatory and degenerative articular disorders such as rheumatoid arthritis and arthrosis, impairments of the hemostatic system such as fibrin deposits, fibrotic changes in the lung such as chronic obstructive pulmonary disease, adult respiratory distress syndrome, fibrin deposits in the eye following eye operations or prevention and/or treatment of scarring.

10. A process for preparing a compound of the formula I or Ia or a physiologically acceptable salt thereof as claimed in one or more of claims 1 to 5, which comprises
a) reacting a compound of the formula II, 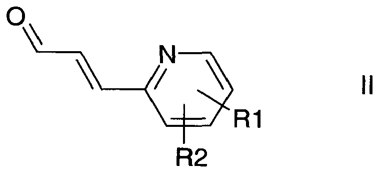 where the radicals R1, R2 are as defined in formula I or Ia, with a compound of the formula III and a compound of the formula IV, 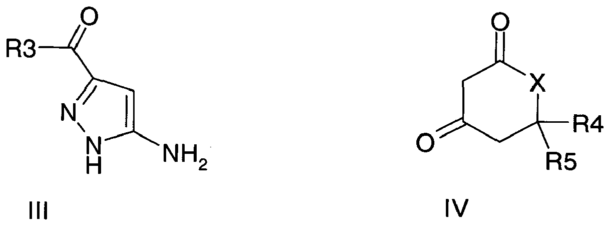 where the radicals R1, R2 are as defined in formula I or Ia, in the presence of a suitable solvent or solvent mixture at 20°C to 120°C to give a compound of the formula I or Ia; or
b) reacting a compound of the formula V, 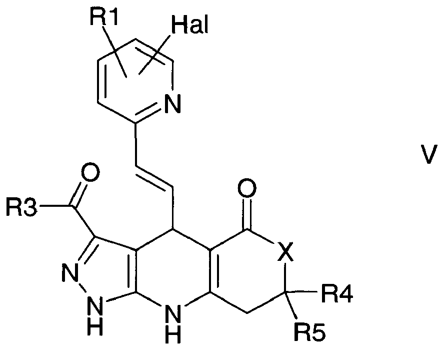 where the radicals X, R1, R3, R4 and R5 are as defined in formula I or Ia, and Hal has the meaning of chlorine, bromine, iodine or triflate, with a compound of the formula R2-B(OH)₂ in the presence of a base and of a suitable metal catalyst in a suitable solvent or solvent mixture to give a compound of the formula I or II; or
c) reacting a compound of the formula I in which X has the meaning of NH with a suitable alkylating agent in the presence of a base and in a suitable inert solvent at room temperature or at elevated temperature to give a compound of the formula I in which X has the meaning of N-R6 and R6 is -(C₁-C₆)-alkyl, -(C₁-C₆)-alkylene-(C₃-C₇)-cycloalkyl or -(C₁-C₆)-alkylenearyl ; or
d) converting a compound of the formula I or Ia in which R3 is -OH, -O-aryl or -O-(C₁-C₆)-alkyl and R1, R2, R4, R5 and X are as defined in formula I or Ia by conventional processes into a compound of the formula I or Ia in which R3 is -NH₂, -NH-(C₁-C₆)-alkyl, -NH-(C₁-C₆) -alkylenearyl, -NH- (C₁-C₆) -alkylene- (C₃-C₇) - cycloalkyl, -NH-(C₁-C₆)-alkylenehetaryl, -N((C₁-C₄)-alkyl)₂ or a cyclic amine which is selected from the group consisting of hexamethyleneimine, morpholine, piperazine, piperidine, pyrrolidine and thiomorpholine, and is attached via the nitrogen atom, where aryl, hetaryl and cyclic amine are in each case unsubstituted or mono-, di- or trisubstituted independently of one another by F, Cl, -OH, - (C₁-C₄)-alkyl, -O-(C₁-C₄)-alkyl, aryl or hetaryl; or
e) fractionating the compound of the formula I or Ia which has been prepared by processes a) to d), or a suitable precursor of the compound of the formula I or Ia which, owing to its chemical structure, occurs in enantiomeric or diastereomeric forms, by salt formation with enantiomerically pure acids or bases, chromatography on chiral stationary phases or derivatization by means of chiral enantiomerically pure compounds such as amino acids, separation of the diastereomers obtained in this way, and elimination of the chiral auxiliary groups, into the pure enantiomers or diastereomers; or
f) either isolating the compound of the formula I or Ia prepared by processes a) to e) in free form or liberating it from a non-physiologically acceptable salt or, in the case where acidic or basic groups are present, converting it into a physiologically acceptable salt.

## Revendications

1. Composé de formule I 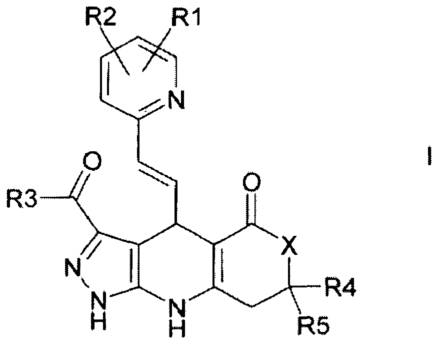 et/ou toutes les formes stéréoisomères ou tautomères du composé de formule I et/ou mélanges de ces formes en un rapport quelconque et/ou sel physiologiquement compatible du composé de formule I, dans laquelle
R1 représente alkyle en (C₁-C₆), aryle, halogène ou hétaryle choisi dans le groupe constitué par thiényle, pyrrolyle, imidazolyle, pyrazolyle, pyridyle, pyrimidinyle, pyrazinyle ou pyridazinyle ; alkyle, aryle et hétaryle étant chacun non substitués ou substitués une, deux ou trois fois indépendamment les uns des autres par F, Cl, Br, CN, alkyle en (C₁-C₄), -0-alkyle en (C₁-C₄) ou -CF₃ ;
R2 représente hydrogène ;
R3 représente -OH, -0-alkyle en (C₁-C₈), -O-alkylène en (C₁-C₆)-NH-C(O)-O-alkyle en (C₁-C₄), -O-alkylène en (C₁-C₆) -NH₂, -NH₂, -NH-alkyle en (C₁-C₈), -NH-alkylène en (C₁-C₆) -aryle, -NH-alkylène en (C₁-C₈)-O-alkyle en (C₁-C₄), -NH-alkyle en (C₁-C₈) -OH, -NH-alkylène en (C₁-C₆) - NH-C(O)-O-alkyle en (C₁-C₄), -NH-alkylène en (C₁-C₆)-NH₂, -NH-alkylène en (C₀-C₆)-cycloalkyle en (C₃-C₇), -NH-alkylène en (C₀-C₆)-hétaryle ou -N(alkyle en (C₁-C₄)₂ ; aryle, cycloalkyle et hétaryle étant chacun non substitués ou substitués une, deux ou trois fois indépendamment les uns des autres par F, Cl, -OH, alkyle en (C₁-C₄), -alkylène en (C₁-C₄)-NH₂, -alkylène en (C₁-C₄) -OH ou -O-alkyle en (C₁-C₄) ;
R4 et R5 sont identiques ou différents et représentent indépendamment l'un de l'autre hydrogène ou alkyle en (C₁-C₆), ou
R4 et R5 forment ensemble avec l'atome C auquel ils sont reliés un radical cycloalkyle en (C₃-C₇) ;
X représente une liaison covalente, CH₂, CH(alkyle en (C₁-C₆)), C(alkyle en (C₁-C₄))₂ ou oxygène ;
le terme « hétaryle » dans les systèmes cycliques de R3 étant compris comme contenant 4 à 15 atomes de carbone, qui se présentent dans un, deux ou trois systèmes cycliques reliés les uns aux autres et qui contiennent, selon la taille des cycles, un, deux, trois ou quatre hétéroatomes identiques ou différents de la série constituée par oxygène, azote ou soufre, ou hétaryle représentant un radical oxétanyle.

2. Composé de formule I selon la revendication 1, et/ou toutes les formes stéréoisomères ou tautomères du composé de formule I et/ou mélanges de ces formes en un rapport quelconque et/ou sel physiologiquement compatible du composé de formule I, dans laquelle
R1 représente alkyle en (C₁-C₆), aryle, halogène ou hétaryle choisi dans le groupe constitué par thiényle, pyrrolyle, imidazolyle, pyrazolyle, pyridyle, pyrimidinyle, pyrazinyle ou pyridazinyle ; alkyle, aryle ou hétaryle étant chacun non substitués ou substitués une, deux ou trois fois indépendamment les uns des autres par F, Cl, Br, CN, alkyle en (C₁-C₄), -0-alkyle en (C₁-C₄) ou -CF₃ ;
R2 représente hydrogène ;
R3 représente -O-alkyle en (C₁-C₆), -O-alkylène en (C₁-C₆)-NH-C(O)-O-alkyle en (C₁-C₄), -O-alkylène en (C₁-C₆) -NH₂, -NH-alkyle en (C₁-C₆), -NH-alkylène en (C₁-C₆)-aryle, -NH-alkylène en (C₁-C₆) -cycloalkyle en (C₃-C₇), - NH-alkylène en (C₁-C₆)-hétaryle ou -N(alkyle en (C₁-C₄))₂ ; aryle, cycloalkyle et hétaryle étant chacun non substitués ou substitués une, deux ou trois fois indépendamment les uns des autres par F, Cl, -OH, -alkylène en (C₁-C₄)-NH₂, -alkylène en (C₁-C₄)-OH ou alkyle en (C₁-C₄) ;
R4 et R5 sont identiques ou différents et représentent indépendamment l'un de l'autre hydrogène ou alkyle en (C₁-C₆), ou
R4 et R5 forment ensemble avec l'atome C auquel ils sont reliés un cycloalkyle en (C₃-C₆) ;
X représente une liaison covalente, CH₂, CH(alkyle en (C₁-C₄)), C(alkyle en (C₁-C₄))₂ ou oxygène ;
le terme « hétaryle » dans les systèmes cycliques de R3 étant compris comme contenant 4 à 15 atomes de carbone, qui se présentent dans un, deux ou trois systèmes cycliques reliés les uns aux autres et qui contiennent, selon la taille des cycles, un, deux, trois ou quatre hétéroatomes identiques ou différents de la série constituée par oxygène, azote ou soufre, ou hétaryle représentant un radical oxétanyle.

3. Composé de formule Ia selon la revendication 1, 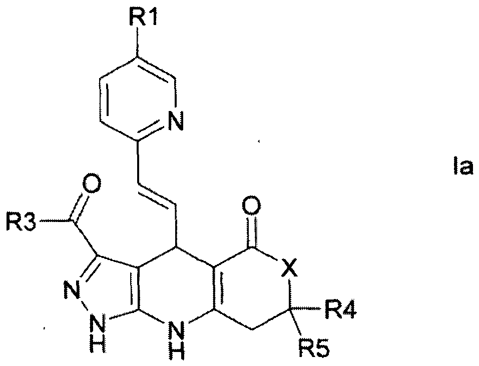 et/ou toutes les formes stéréoisomères ou tautomères du composé de formule Ia et/ou mélanges de ces formes en un rapport quelconque et/ou sel physiologiquement compatible du composé de formule Ia, dans laquelle
R1 représente hydrogène, alkyle en (C₁-C₄), phényle, Cl, Br ou hétaryle choisi dans le groupe constitué par thiényle, pyrrolyle, imidazolyle, pyrazolyle, pyridyle, pyrimidinyle, pyrazinyle ou pyridazinyle ; phényle et hétaryle étant chacun non substitués ou substitués une ou deux fois indépendamment l'un de l'autre par F, Br, Cl, CN, alkyle en (C₁-C₄), -O-alkyle en (C₁-C₄) ou -CF₃ ; R3 représente -OH, -O-alkyle en (C₁-C₆), -O-alkylène en (C₁-C₆)-NH-C(O)-O-alkyle en (C₁-C₄), -O-alkylène en (C₁-C₆)-NH₂, -NH-alkyle en (C₁-C₄), -NH-benzyle ou -NH-méthylène-cycloalkyle en (C₃-C₆) ; cycloalkyle étant non substitué ou substitué une fois par alkyle en (C₁-C₄), -alkylène en (C₁-C₄) -NH₂ ou -CH₂-OH ;
R4 et R5 sont identiques ou différents et représentent indépendamment l'un de l'autre hydrogène ou alkyle en (C₁-C₄), ou
R4 et R5 forment ensemble avec l'atome C auquel ils sont reliés un cyclopropyle, cyclobutyle, cyclopentyle ou cyclohexyle ;
X représente une liaison covalente, CH₂, C(alkyle en (C₁-C₄))₂ ou oxygène.

4. Composé de formule Ia selon la revendication 3, et/ou toutes les formes stéréoisomères ou tautomères du composé de formule Ia et/ou mélanges de ces formes en un rapport quelconque et/ou sel physiologiquement compatible du composé de formule Ia, dans laquelle
R1 représente alkyle en (C₁-C₄), phényle, Cl ou Br ; phényle étant non substitué ou substitué une, deux ou trois fois indépendamment par F, Cl, Br, CN, alkyle en (C₁-C₄), -O-alkyle en (C₁-C₄) ou -CF₃ ;
R3 représente -OH, -O-alkyle en (C₁-C₄), -O-alkylène en (C₁-C₆)-NH-C(O)-O-alkyle en (C₁-C₄), -O-alkylène en (C₁-C₆)-NH₂, -NH-alkyle en (C₁-C₃) ou -NH-méthylène-cycloalkyle en (C₃-C₆) ; cycloalkyle étant non substitué ou substitué une fois par alkyle en (C₁-C₄), -alkylène en (C₁-C₄)-NH₂ ou -CH₂-OH ;
R4 et R5 sont identiques ou différents et représentent indépendamment l'un de l'autre hydrogène ou alkyle en (C₁-C₄), ou
R4 et R5 forment ensemble avec l'atome C auquel ils sont reliés un cyclobutyle ou cyclopentyle ;
X représente une liaison covalente, CH₂, C(alkyle en (C₁-C₄))₂ ou oxygène.

5. Composé de formule I ou Ia selon les revendications 1 à 4, et/ou toutes les formes stéréoisomères ou tautomères du composé de formule I ou Ia et/ou mélanges de ces formes en un rapport quelconque et/ou sel physiologiquement compatible du composé de formule I ou Ia, le composé de formule I ou Ia étant choisi dans le groupe constitué par :
le cyclopropylméthylamide de l'acide 4-{(E)-2-[5-(3-fluoro-phényl)-pyridin-2-yl]-vinyl}-7,7-diméthyl-5-oxo-4,5,6,7,8,9-hexahydro-1H-pyrazolo[3,4-b]quinoline-3-carboxylique,
l'isopropylamide de l'acide 7,7-diméthyl-4-[(E)-2-(6-méthyl-pyridin-2-yl)-vinyl]-5-oxo-4,5,6,7,8,9-hexahydro-1H-pyrazolo[3,4-b]quinoline-3-carboxylique,
l'isopropylamide de l'acide 4-{(E)-2-[5-(3-fluoro-phényl)-pyridin-2-yl]-vinyl}-7,7-diméthyl-5-oxo-4,5,6,7,8,9-hexahydro-1H-pyrazolo[3,4-b]quinoline-3-carboxylique,
le cyclopropylméthylamide de l'acide 4-{(E)-2-[5-(3-fluoro-phényl)-pyridin-2-yl]-vinyl}-6,6-diméthyl-5-oxo-4,5,6,7,8,9-hexahydro-1H-pyrazolo[3,4-b]quinoline-3-carboxylique,
le cyclopropylméthylamide de l'acide 4-{(E)-2-[5-(3-fluoro-phényl)-pyridin-2-yl]-vinyl}-7-isopropyl-5-oxo-4,5,6,7,8,9-hexahydro-1H-pyrazolo[3,4-b]quinoline-3-carboxylique,
le cyclopropylméthylamide de l'acide 4-[(E)-2-(5-bromo-pyridin-2-yl)-vinyl]-7,7-diméthyl-5-oxo-4,5,6,7,8,9-hexahydro-1H-pyrazolo[3,4-b]quinoline-3-carboxylique,
l'isopropylamide de l'acide 4-[(E)-2-(5-bromo-pyridin-2-yl)-vinyl]-7,7-diméthyl-5-oxo-4,5,6,7,8,9-hexahydro-1H-pyrazolo[3,4-b]quinoline-3-carboxylique,
l'isopropylamide de l'acide 4-{(E)-2-[5-(3-fluoro-phényl)-pyridin-2-yl]-vinyl}-7-méthyl-5-oxo-4,5,6,7,8,9-hexahydro-1H-pyrazolo[3,4-b]quinoline-3-carboxylique,
le cyclopropylméthylamide de l'acide 4-{(E)-2-[5-(3-fluoro-phényl)-pyridin-2-yl]-vinyl}-7-méthyl-5-oxo-4,5,6,7,8,9-hexahydro-1H-pyrazolo[3,4-b]quinoline-3-carboxylique,
l'ester éthylique de l'acide 4-{(E)-2-[5-(3-fluoro-phényl)-pyridin-2-yl]-vinyl}-7,7-diméthyl-5-oxo-4,5,6,7,8,9-hexahydro-1H-pyrazolo[3,4-b]quinoline-3-carboxylique et
le (2,2-diméthyl-propyl)amide de l'acide 4-{(E)-2-[5-(3-fluoro-phényl)-pyridin-2-yl]-vinyl}-7,7-diméthyl-5-oxo-4,5,6,7,8,9-hexahydro-1H-pyrazolo[3,4-b]quinoline-3-carboxylique.

6. Médicament, **caractérisé par** une teneur efficace en au moins un composé de formule I ou Ia ou un sel physiologiquement compatible de celui-ci selon une ou plusieurs des revendications 1 à 5, conjointement avec un véhicule pharmaceutiquement approprié et physiologiquement compatible, un additif et/ou d'autres agents actifs et adjuvants.

7. Composé de formule I ou Ia ou sel physiologiquement compatible de celui-ci selon une ou plusieurs des revendications 1 à 5, destiné à une utilisation en tant que produit pharmaceutique.

8. Composé de formule I ou Ia ou sel physiologiquement compatible de celui-ci selon une ou plusieurs des revendications 1 à 5, destiné à une utilisation pour la prophylaxie, la prévention secondaire et le traitement de toutes les maladies qui accompagnent les thromboses, les embolies, l'hypercoagulabilité, les modifications fibrotiques ou les maladies inflammatoires.

9. Composé de formule I ou Ia ou sel physiologiquement compatible de celui-ci selon une ou plusieurs des revendications 1 à 5, destiné à une utilisation selon la revendication 8, **caractérisé en ce qu'**il s'agit d'un infarctus du myocarde, d'une angine de poitrine et d'autres formes du syndrome coronarien aigu, de l'attaque cérébrale aigue ou de sa prévention secondaire, des maladies vasculaires périphériques, de la thrombose veineuse profonde, de l'embolie pulmonaire, des évènements emboliques ou thrombotiques causés par des arythmies cardiaques, des évènements cardiovasculaires tels que les resténoses après une revascularisation et une angioplastie et des interventions semblables telles que des implantations de stents et des opérations de pontage, de la réduction du risque de thrombose après des interventions chirurgicales telles que des opérations du genou et des opérations de la hanche, des interventions qui conduisent à un contact du sang avec des surfaces étrangères telles que pour les patients recevant une dialyse et les patients munis de sondes à demeure, de la coagulation intravasculaire disséminée, du sepsis et d'autres évènements intravasculaires qui accompagnent une inflammation, de l'athérosclérose, du diabète et du syndrome métabolique et de leurs conséquences, de la croissance de tumeurs et de la métastase de tumeurs, des maladies inflammatoires et dégénératives des articulations, telles que la polyarthrite rhumatoïde et l'arthrose, des troubles du système hémostatique tels que les dépôts de fibrine, des modifications fibrotiques des poumons, telles que la pneumopathie obstructive chronique, du syndrome de détresse respiratoire de l'adulte, des dépôts de fibrine de l'oeil après des opérations de l'oeil, ou de la prévention et/ou du traitement de la formation de cicatrices.

10. Procédé de fabrication d'un composé de formule I ou Ia ou d'un sel physiologiquement compatible de celui-ci selon une ou plusieurs des revendications 1 à 5, **caractérisé en ce que**
a) un composé de formule II 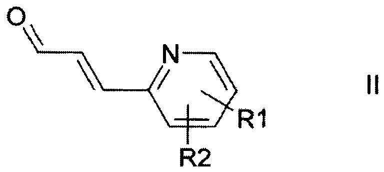 dans laquelle les radicaux R1, R2 sont tels que définis pour la formule I ou Ia, est mis en réaction avec un composé de formule III et un composé de formule IV 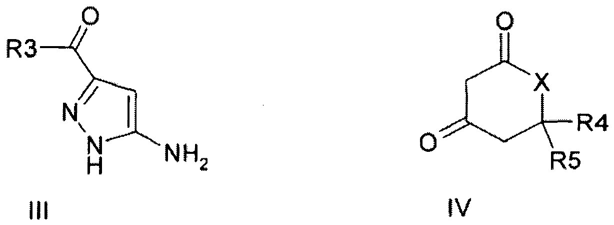 dans lesquelles les radicaux R1, R2 sont tels que définis pour la formule I ou Ia, en présence d'un solvant ou mélange de solvants approprié, à une température de 20 °C à 120 °C, pour former un composé de formule I ou Ia ; ou
b) un composé de formule V 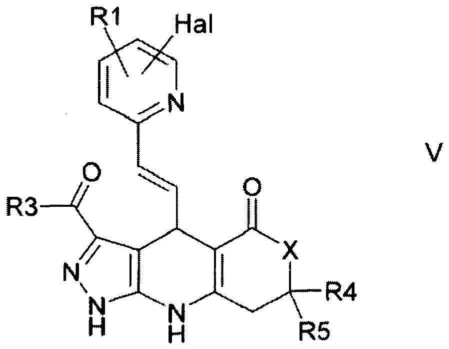 dans laquelle les radicaux X, R1, R3, R4 et R5 sont tels que définis pour la formule I ou Ia, et Hal a la signification chlore, brome, iode ou triflate, est mis en réaction avec un composé de formule R2-B(OH)₂ en présence d'une base et d'un catalyseur métallique approprié dans un solvant ou mélange de solvants approprié pour former un composé de formule I ou II ; ou
c) un composé de formule I, dans laquelle X a la signification NH, est mis en réaction avec un agent d'alkylation approprié en présence d'une base et dans un solvant inerte approprié, à température ambiante ou à une température élevée, pour former un composé de formule I, dans laquelle X a la signification N-R6 et R6 signifie alkyle en (C₁-C₆), -alkylène en (C₁-C₆)-cycloalkyle en (C₃-C₇) ou -alkylène en (C₁-C₆) -aryle ; ou
d) un composé de formule I ou Ia, dans laquelle R3 représente -OH, -O-aryle ou -O-alkyle en (C₁-C₆), et R1, R2, R4, R5 et X sont tels que définis pour la formule I ou Ia, est mis en réaction selon des procédés usuels pour former un composé de formule I ou Ia, dans laquelle R3 représente -NH₂, -NH-alkyle en (C₁-C₆), -NH-alkylène en (C₁-C₆) -aryle, -NH-alkylène en (C₁-C₆) - cycloalkyle en (C₃-C₇), -NH-alkylène en (C₁-C₆) -hétaryle, -N(alkyle en (C₁-C₄))₂ ou une amine cyclique choisie dans le groupe constitué par l'hexaméthylène-imine, la morpholine, la pipérazine, la pipéridine, la pyrrolidine et la thiomorpholine, et reliée par l'atome d'azote ; aryle, hétaryle et l'amine cyclique étant chacun non substitués ou substitués une, deux ou trois fois indépendamment les uns des autres par F, Cl, -OH, alkyle en (C₁-C₄), -O-alkyle en (C₁-C₄), aryle ou hétaryle ; ou
e) le composé de formule I ou Ia fabriqué par les procédés a) à d), ou un précurseur approprié du composé de formule I ou Ia, qui se présente en raison de sa structure chimique sous des formes énantiomères ou diastéréomères, est séparé par formation d'un sel avec des acides ou bases énantiomériquement purs, chromatographie sur des phases stationnaires chirales ou dérivation avec des composés chiraux énantiomériquement purs tels que des acides aminés, séparation des diastéréomères ainsi obtenus et clivage des groupes auxiliaires chiraux en les énantiomères ou diastéréomères purs ; ou
f) le composé de formule I ou Ia fabriqué par les procédés a) à e) est isolé sous forme libre ou libéré d'un sel physiologiquement incompatible ou, lors de la présence de groupes acides ou basiques, transformé en un sel physiologiquement compatible.
